# EUROPÄISCHE PATENTSCHRIFT

(11) **EP 1 362 118 B1**
(45) Veröffentlichungstag und Bekanntmachung des Hinweises auf die Patenterteilung: **11.10.2006**
(21) Anmeldenummer: 02719874.6
(22) Anmeldetag: 20.02.2002
(51) Int. Cl.: C12P 13/02, A23K 1/00

(54) **VERFAHREN ZUR HERSTELLUNG VON D-PANTOTHENSÄURE UND/ODER DEREN SALZE ALS ZUSATZ ZU TIERFUTTERMITTELN**
METHOD FOR PRODUCING D-PANTOTHENIC ACID AND/OR SALTS THEREOF AS AN ADDITIVE FOR ANIMAL FEED
PROCEDE POUR LA PRODUCTION D'ACIDE D-PANTOTHENIQUE OU DE SES SELS COMME ADDITIF A DES ALIMENTS POUR ANIMAUX

(30) Priorität: 21.02.2001 DE 10108223
(43) Veröffentlichungstag der Anmeldung: 19.11.2003
(73) Patentinhaber: BASF Aktiengesellschaft, 67056 Ludwigshafen (DE)
(72) Erfinder: BALDENIUS, Kai-Uwe, 67063 Ludwigshafen (DE); BECK, Christine, 68167 Mannheim (DE); FISCHER, Andreas, 67067 Ludwigshafen (DE); HARZ, Hans-Peter, 67373 Dudenhofen (DE); LOHSCHEIDT, Markus, 68199 Mannheim (DE); LEEMANN, Martin, 64625 Bensheim (DE)
(74) Vertreter: Fitzner, Uwe
(86) Internationale Anmeldenummer: PCT/EP2002/001766
(87) Internationale Veröffentlichungsnummer: WO 2002/066666

(56) Entgegenhaltungen:
- EP-A- 0 493 060
- EP-A- 0 590 857
- EP-A- 1 006 192
- EP-A- 1 050 219
- WO-A-01/21772
- WO-A-02/24001
- GB-A- 562 267
- BAIGORI M ET AL.: "Isolation and characterization of Bacillus subtilis mutants blocked in the synthesis of pantothenic acid" JOURNAL OF BACTERIOLOGY, Bd. 173, Nr. 13, Juli 1991 (1991-07), Seiten 4240-4242, XP001002216 ISSN: 0021-9193
- DATABASE EMBL [Online] EMBL; EMBL:BSYPIA, ID BSYPIA, AC L47709, 23. Januar 1996 (1996-01-23) HENNER D ET AL.: "Bacillus subtilis (clone YAC15-6B) ypiABF genes, qcrABC genes, ypjABCDEFGHI genes, birA gene, panBCD genes, dinG gene, ypmB gene, aspB gene, asnS gene, dnaD gene, nth gene and ypoC gene, complete cds" Database accession no. L47709 XP002171539
- BEGLEY T P ET AL.: "The biosynthesis of Coenzyme A in bacteria" VITAMINS AND HORMONES, ACADEMIC PRESS, NEW YORK, US, Bd. 61, Februar 2001 (2001-02), Seiten 157-171, XP008004305 ISSN: 0083-6729

## Beschreibung

Die vorliegende Erfindung betrifft ein verbessertes Verfahren zur Herstellung von D-Pantothensäure und/oder deren Salze und die Verwendung als Zusatz zu Tierfuttermitteln.

Als ein Ausgangsprodukt der Biosynthese von Coenzym A ist D-Pantothenat in der Pflanzen- und Tierwelt weit verbreitet. Im Gegensatz zum Menschen, der die Pantothensäure in ausreichenden Mengen über die Nahrung zu sich nimmt, sind jedoch sowohl für Pflanzen als auch für Tiere häufig Mangelerscheinungen für D-Pantothenat beschrieben. Die Verfügbarkeit von D-Pantothenat ist daher von großem wirtschaftlichen Interesse, insbesondere in der Tierfutterindustrie.

Herkömmlicher Weise erfolgt die Herstellung von D-Pantothenat durch chemische Synthese aus D-Pantölacton und Calcium-β-Alaninat (Ullmann's Encyclopedia of Industrial Chemistry, 6^{th} edition, 1999, electronic release, Kapitel "Vitamins"). Zur Bereitstellung von D-Pantolacton ist eine aufwendige, klassische Racematspaltung über diastereomere Salze erforderlich. Das aus der chemischen Synthese resultierende Verkaufsprodukt ist meist das Calciumsalz der D-Pantothensäure Calcium-D-Pantothenat.

Gegenüber der chemischen Synthese liegt der Vorteil biotechnologischer Herstellungsverfahren mit Mikroorganismen in der selektiven (enantiomerenreinen) Bereitstellung der für den höheren Organismus verwertbaren D-Form der Pantothensäure. Eine aufwendige Racematspaltung, wie sie bei der chemischen Synthese erforderlich ist, entfällt somit.

Fermentative Verfahren zur Herstellung von D-Pantothensäure mit Mikroorganismen sind zahlreich bekannt, so u.a. aus EP 0 590 857, WO 96/33283, US 6,013,492, WO 97/10340, DE 198 46 499, EP 1 001 027, EP 1 006 189, EP 1 006 192 und EP 1 006 193.

So beschreiben EP 1 006 189 und EP 1 001 027 Verfahren zur Herstellung von Pantothenat bei dem in der Fermentationslösung ein Gehalt von höchstens 1 g/l D-Pantothensäure erreicht wird. Solche geringen Pantothensäure-Gehalte in der Fermentationslösung, also von weniger als 10 Gew.-% bezogen auf den Feststoffgehalt sind jedoch für eine wirtschaftliche Herstellung von D-Pantothensäure enthaltenden Tierfuttersupplementen ungeeignet. Ein weiterer Nachteil bei den bislang beschriebenen Verfahren ist, daß die Isolierung des Produkts aus dem Fermentationsmedium zahlreiche aufwendige Aufarbeitungsschritte erfordert. Ein wirtschaftliches Herstellungsverfahren für den großtechnischen Maßstab ist nicht offenbart.

In der Offentegungsschrift DE 100 16 321 ist ein Fermentationsverfahren zur Herstellung eines D-Pantothensäure-haltigen Tierfuttersupplements beschrieben. Ein wesentlicher Nachteil dieses Verfahrens ist jedoch, wie auch bei den oben angeführten fermentativen Verfahren zur D-Pantothensäure-Herstellung, daß dem Mikroorganismus über das Fermentationsmedium die Pantothensäure-Vorstufe β-Alanin zwingend zugeführt werden muß, um wirtschaftliche Ausbeuten des gewünschten Produktes zu erhalten.

Ferner beschreiben US 6,013,492 und WO 96/332839 die Aufarbeitung der D-Pantothensäure aus der Fermentationslösung durch Abfiltem unlöslicher Bestandteile (z. B. Zellmaterial) vom Kulturmedium, eine Adsorption des Filtrats an Aktivkohle, eine sich anschließende Elution der D-Pantothensäure mit einem organischen Lösungsmittel, bevorzugt Methanol, eine Neutralisation mit Calciumhydroxid und eine abschließende Kristallisation von Calcium-D-Pantothenat. Wesentliche Nachteile sind die bei der Kristallisation auftretenden Wertproduktverluste sowie die Verwendung eines organischen Lösungsmittels, das nur schwer aus dem Produkt zu entfernen ist und eine aufwendige Lösungsmittelrückgewinnung erforderlich macht.

Die EP 0 590 857 beschreibt ein Fermentationsverfahren zur Herstellung von D-Pantothensäure, bei dem die Kultivierung eines Mikroorganismus die Zufütterung von β-Alanin zwingend erfordert. Die Fermentationslösung wird zur Abtrennung der Biomasse filtriert, dann über einen Kationenaustauscher und anschließend über einen Anionenaustauscher geleitet, im Anschluß daran mit Calciumhydroxid neutralisiert, eingedampft, mit Aktivkohle versetzt, nochmals filtriert und unter Zusatz von Methanol und Calciumchlorid kristallisiert. Das resultierende Calciumpantothenat-haltige Produkt enthält neben D-Pantothensäure in Form des Caliumssalzes noch Calciumchlorid in einem Molverhältnis von 1:1. Zur Reduzierung des Calciumchloridgehaltes ist eine Elektrodialyse mit anschließender Sprühtrocknung erforderlich. Dieses Verfahren hat den Nachteil, wegen der Vielzahl aufwendiger Verfahrensschritte und der Verwendung organischer Lösungsmittel weder ökonomisch noch ökologisch zu sein.

Aufgabe der vorliegenden Erfindung ist die Bereitstellung eines D-Pantothensäure und/oder deren Salze enthaltendes Tierfuttersupptement sowie dessen Herstellung durch ein verbessertes Verfahren zur Herstellung von D-Pantothensäure und/oder deren Salzen, das die zuvor genannten Nachteile nicht aufweist. Hierbei ist aus wirtschaftlichen Gründen ein Verfahren wünschenswert, bei dem eine Zufütterung von β-Alanin drastisch reduziert oder überhaupt nicht erforderlich ist. Ferner ist die Herstellung von D-Pantothensäure in Form ihrer zweiwertigen Salze und hierbei vor allem der Erdalkali-Salze wünschenswert, da die zweiwertigen Salze weniger hygroskopische Eigenschaften aufweisen als einwertige Salze der Pantothensäure und für die weitere Verwendung, z. B. als Tierfuttersupplement, somit weniger stark zu Verklumpungen neigen. Diese Aufgabe wird durch die vorliegende Erfindung in vorteilhafter Weise gelöst.

Gegenstand der vorliegenden Erfindung ist ein Verfahren zur Herstellung von D-Pantothensäure und/oder deren Salze, dadurch gekennzeichnet, daß
a) wenigstens ein D-Pantothensäure produzierender Mikroorganismus eingesetzt wird, dessen Pantothensäure-(pan)- und/oder Isoleucin/Valin-(ilv)-Biosynthese dereguliert ist und der wenigstens 2 g/l an Salzen der D-Pantothensäure durch Fermentation in einem Kulturmedium bildet, wobei dem Kulturmedium 0 - 20 g/l freies β-Alanin und/oder β-Alanin-Salz zugeführt wird,
b) die D-Pantothenat enthaltende Fermentationslösung durch Anlegen eins elektrischen Feldes über eine oder mehrere ionenselektive Membran(en) geleitet wird, wobei niedermolekulare Ionen aus der D-Pantothenat-haltigen Lösung entfernt werden,
c) die in der Lösung enthaltene freie D-Pantothensäure durch die Zugabe von Calcium- und/oder Magnesiumbase auf einen pH-Wert von 5-10 eingestellt wird, wobei eine Lösung erhalten wird, die Calcium- und/oder Magnesium-Pantothenat enthält und
d) die Calcium- und/oder Magnesium-Pantothenat enthaltende Lösung einer Trocknung und/oder Formulierung unterzogen wird.

In einer Variante des erfindungsgemäßen Verfahrens wird in Schritt c) oder d) eine Suspension erhalten oder vorgelegt, die Calcium- und/oder Magnesium-Pantothenat enthält.

Ferner kann die erfindungsgemäß in Schritt a) erfolgende Fermentation nach an sich bekannten Vorgehensweisen im batch-, fed-batch- oder repeated-fed-batch-Betrieb oder bei kontinuierlicher Prozeßführung durchgeführt werden. Zur Neutralisation der entstehenden Pantothensäure werden hierbei übliche Puffersysteme, wie z. B. Phosphat-Puffer mit NaOH, KOH oder Ammoniak benutzt.

In weiteren Varianten des erfindungsgemäßen Verfahrens werden in Schritt a) wenigstens 10 g/l, bevorzugt wenigstens 20 g/l, besonders bevorzugt wenigstens 40 g/l, höchst besonders bevorzugt wenigstens 60 g/l und insbesondere wenigstens 70 g/l an Salzen der D-Pantothensäure durch Fermentation im Kulturmedium gebildet.

Erfindungsgemäß ist unter der Formulierung "produzieren" zu verstehen, daß der Mikroorganismus größere Mengen D-Pantothensäure und/oder deren Salze synthetisieren kann, als für den eigenen Stoffwechselbedarf erforderlich sind. In einer erfindungsgemäß vorteilhaften Variante liegt die synthetisierte Menge an D-Pantothensäure und/oder deren Salze nicht zellintem vor, sondern wird idealerweise vollständig aus dem Mikroorganismus in das Kulturmedium abgegeben. Dies Ausschleusung kann aktiv oder passiv nach an sich bekannten Mechanismen erfolgen.

Erfindungsgemäß werden als D-Pantothensäure produzierende Mikroorganismen Pilze, Hefen und/oder Bakterien eingesetzt. Erfindungsgemäß werden bevorzugt Pilze wie beispielsweise Mucor oder Hefen, wie z. B, Saccharomyces oder Debaromyces und hierbei bevorzugt Saccharaomyces cerevisiae eingesetzt. Vorteilhaft werden erfindungsgemäß coryneforme Bakterien oder Bacillaceae verwendet. Erfindungsgemäß umfaßt sind bevorzugt z. B. Bakterien der Gattungen Corynebacterium, Escherichia, Bacillus, Arthrobacter, Bevibacterium, Pseudomonas, Salmonella, Klebsiella, Proteus, Acinetobacter oder Rhizobium. Besonders bevorzugt sind hierbei beispielsweise Corynebacterium glutamicum, Brevibacterium breve oder Bacillus subtilis, B. licheniformis, B. amyloliquefaciens, B. cereus, B. lentimorbus, B. lentus, B. firmus, B.pantothenticus, B. circulans, B. coagulans, B. megaterium, B. pumilus, B. thuringiensis, B. brevis, B. stearothermophilus und andere Bacillusarten der Gruppe 1, die durch ihre 16sRNA charakerisiert sind oder Actinum mycetalis. Diese Aufzählung dient der Erläuterung und ist keinesfalls limitierend für die vorliegende Erfindung.

Darüber hinaus umfaßt die vorliegende Erfindung auch den Einsatz genetisch veränderter Mikroorganismen zur erfindungsgemäßen Herstellung eines Tierfuttersupplements enthaltend freie D-Pantothensäure und/oder deren Salze. Solche genetisch veränderten Mikroorganismen können beispielsweise durch chemische Mutagenese und anschließende Selektion durch eine geeignetes "Screeningverfahren" isoliert werden. Erfindungsgemäß sind auch sogenannte "Produktionsstämme" umfaßt, die zur Herstellung des Produktes im Sinne der vorliegenden Erfindung geeignet sind und genetische Veränderungen hinsichtlich des Stoffwechselflusses in Richtung D-Pantothensäure aufweisen, wobei auch Veränderungen hinsichtlich der Ausschleusung von D-Pantothensäure und/oder deren Salzen über die Zellmembran inbegriffen sind. Dies kann z. B. durch Veränderungen an Schlüsselpositionen in relevanten Stoffwechsel-Biosynthesewegen des eingesetzten Mikroorganismus erreicht werden.

Denkbar ist auch der Einsatz transgener Mikroorganismen, die aus der Übertragung homologer und/oder heterologer Nukleotidsequenzen resultieren, welche zur Synthese des gewünschten Produktes erforderlich sind oder förderlich sein können. Hierbei ist die Überexpression und/oder Deregulation ein oder mehrerer Gene einzeln und/oder in Kombination, lokalisiert im Genom und/oder auf einem Vektor, denkbar.
Derartige transgene Mikroorganismen können in vorteilhafter Weise zusätzliche Kopien und/oder genetisch veränderte Gene ausgewählt aus der Gruppe von panB, panC, panD, panE und/oder deren Kombinationen und/oder sogar Organisationseinheiten, wie das panBCD-Operon enthalten. Ferner können weitere Stoffwechselwege, wie z.B. der Isoleucin-Valin-Biosyntheseweg in den Mikroorganismen vorteilhaft manipuliert sein, wie beispielsweise in der EP 1 006 189, EP 1 006 192, EP 1 006 193 oder EP 1 001 027 beschrieben ist. Dadurch werden verzweigtkettige Voriäufersubstanzen der Pantothensäure-Biosynthese vermehrt zur Verfügung gestellt. Vorteilhaft werden gegebenenfalls die Gene für diesen Biosyntheseweg d.h. ilvB, ilvN, ilvC und/oder ilvD überexprimiert.
Darüber hinaus sind genetische Veränderungen der Aspartat-α-Decarboxylase (panD), z.B. durch Überexpression und/oder Deregulation, in dem eingesetzten D-Pantothensäure produzierenden Mikroorganismus erfindungsgemäß umfaßt.

Unter der Formulierung "Deregulation" ist erfindungsgemäß folgendes zu verstehen: Veränderung oder Modifikation mindestens eines Gens, das für ein Enzym in einem biosynthetischen Stoffwechselweg kodiert, so daß die Aktivität des Enzyms in dem Mikroorganismus verändert oder modifiziert ist. Bevorzugt ist, daß mindestens ein Gen, das für ein Enzym eines biosynthetischen Stoffwechslewegs kodiert, derart verändert ist, daß das Genprodukt verstärkt gebildet wird oder eine gesteigerte Aktivität aufweist. Der Begriff "deregulierter Stoffwechselweg" schließt auch einen biosynthetischen Stoffwechselweg mit ein, in den mehr als ein Gen, das für mehr als ein Enzym kodiert, so verändert oder modifiziert ist, daß die Aktivitäten von mehr als einem Enzym verändert oder modifiziert sind.
Veränderungen oder Modifikationen können beinhalten, sind aber nicht beschränkt auf: Entfernen des endogenen Promotors oder regulatorischer Elemente; Einfügen starker Promotoren, induzierbarer Promotoren oder mehrerer Promotoren gleichzeitig; Entfernen regulatorischer Sequenzen, so daß die Expression des Genprodukts verändert ist; Änderung der chromosomalen Lage des Gens; Veränderung der DNA-Sequenz in der Nähe des Gens oder innerhalb des Gens wie z. B. der ribosomalen Bindungsstelle (RBS); Erhöhung der Kopienzahl des Gens im Genom oder durch Einbringung von Plasmiden verschiedener Kopienzahl; Modifikation von Proteinen (z.B. regulatorischen Proteinen, Suppressoren, Enhancem, trankriptionellen Aktivatoren, und ähnliche), die bei der Transkription des Gens und/oder bei der Translation zum Genprodukt eine Rolle spielen. Dazu zählen auch alle anderen Möglichkeiten zur Deregulation der Expression von Genen die Stand der Technik sind wie z. B. die Verwendung von Antisense Oligonukleotiden, oder die Blockierung von Repressor Proteinen.
Deregulation kann auch Veränderungen in der kodierenden Region von Genen beinhalten, die z. B. zu Aufhebung von feedback Regulation in dem Genprodukt oder zu einer größeren oder kleineren spezifischen Aktivität des Genprodukts führen.

Darüber hinaus sind gentechnische Veränderungen an Enzymen erfindungsgemäß vorteilhaft, die den Abfluß von Vorstufen der Pantothensäure und/oder den Fluß der Pantothensäure zu Coenzym A beeinflussen. Beispielhaft für solche Enzyme kodierende Gene sind: alsD, avtA, ilvE, ansB, coaA, coaX und andere mehr. Diese Aufzählung dient der Erläuterung und ist keinesfalls limitierend für die vorliegende Erfindung.
Weiterhin sind gentechnische Veränderungen vorteilhaft, welche die zelluläre Bereitstellung von Cofaktoren (z. B. von Methylentetrahydrofolat, Redoxäquivalenten u. ä.) in für die Pantothensäure-Produktion optimaler Menge sichern.

In vorteilhafter Weise liegt so β-Alanin bereits in den Zellen in erhöhten Konzentrationen gegenüber entsprechend nicht-genetisch veränderten Mikroorganismen vor und muß somit nicht als Precursor dem Kulturmedium zugesetzt werden, wie es z. B. in EP-A-0 590 857 erforderlich ist. Vorteilhaft sind Mikroorganismen, deren Pantothensäure-(pan)- und/oder Isoleucin-Valin-(ilv)-Biosynthese und/oder Asparat-α-Decarboxylase (panD) dereguliert ist. Weiterhin ist eine zusätzliche Überexpression der Ketopanthoat-Reduktase (panE) in den Mikroorganismen von Vorteil.

Weiterhin erfindungsgemäß von Vorteil ist, wenn gegebenenfalls das coaA-Gen, das für die Synthese von CoenzymA erforderlich ist, in seiner Aktivität emiedrigt ist oder (beispielsweise in Bacillus-Arten) ganz ausgeschaltet ist. Bacillus enthält nämlich neben coaA ein weiteres Gen für diese enzymatische Funktion (= coaX). Auch die Aktivität dieses Gens coaX oder des korrespondierenden Enzyms kann verändert, bevorzugt emiedrigt, oder sogar deletiert werden, sofern coaA selbst noch eine ausreichende, wenn auch emiedrigte Enzymaktivität aufweist, d.h. die Enzymaktivität von coaA nicht ganz verloren gegangen ist. Neben der Überexpression der verschiedenen Gene ist auch eine genetische Manipulation der Promotorregionen dieser Gene in der Art vorteilhaft, daß diese Manipulation zu einer Überexpression der Genprodukte führt.

In einer Ausführungsvariante der vorliegenden Erfindung finden die gemäß der Anlage (WO 01/21772) beschriebenen Bakterienstämme, wie z.B. Bacillus subtilis PA 824 und/oder Derivate davon Einsatz. In einer bevorzugten Ausführungsvariante findet erfindungsgemäß der Mikroorganismus Bacillus subtilis PA 668, wie gemäß der Anlage (US 2004/0091979) beschrieben, Einsatz in dem erfindungsgemäßen Verfahren. Diese Stämme Bacillus subtilis PA 824 und PA 668 wurden wie folgt hergestellt:
Ausgehend von dem Stamm *Bacillus subtilis* 168 (Marburg Stamm ATCC 6051), der den Genotyp *trpC2* (Trp⁻) ausweist, wurde über Transduktion des Trp⁺ Markers (aus dem *Bacillus subtilis* Wildtyp W23) der Stamm PY79 erzeugt. In den Stamm PY79 wurden durch klassische gentechnische Methoden (wie z. B. in Harwood, C.R. and Cutting, S.M. (editors), Molecular Biological Methods for *Bacillus* (1990) John Wiley & Sons, Ltd., Chichester, England beschrieben) ΔpanB und ΔpanE1 Mutationen eingebracht.
Der resultierende Stamm wurde mit genomischer DNA des *Bacillus subtilis* Stammes PA221 (Genotyp P₂₆*panBCD, trpC2* (Trp⁻)) und genomischer DNA des *Bacillus subtilis* Stammes PA303 (Genotyp P₂₆*panE1*) transformiert. Der resultierende Stamm PA327 hat den Genotyp P₂₆*panBCD,* P₂₆*panE1* und ist Tryptophan auxotroph (Trp⁻). Mit dem *Bacillus subtilis* Stamm PA327 wurde in 10 ml Kulturen mit SVY-Medium (25 g/L Difco Veal Infusion Broth, 5 g/L Difco Yeast Extract, 5 g/L Na-Glutamat, 2,7 g/L Ammoniumsulfat auf 740 ml Wasser auffüllen, autoklavieren, anschließend Zugabe von 200 ml 1 M Kaliumphosphat, pH 7,0 und 60 ml 50% steriler Glucoselösung), das mit 5 g/L β-Alanin und 5 g/L α-Ketoisovalerat supplementiert war, Pantothensäure-Titer von bis zu 3,0 g/L (24 h) erreicht.
Die Herstellung des *Bacillus subtilis* Stammes PA221 (Genotyp P₂₆*panBCD, trpC2* (Trp⁻)) ist in folgendem Abschnitt beschrieben :
Durch klassische gentechnische Methoden wurde mit Hilfe der Sequenzinformation des *panBCD* Operons von *E. coli* (siehe. Merkel et al., FEMS Microbiol. Lett., 143, 1996:247-252) ausgehend von einer *Bacillus subtilis* GP275 Plasmid-Bibliothek das *panBCD* Operon von *Bacillus* kloniert. Zur Klonierung wurde der *E. coli* Stamm BM4062 (bir^{ts}) und die Information, daß das *Bacillus* Operon in der Nähe des *birA* Gens liegt, verwendet. Das *panBCD* Operon wurde in ein in *E. coli* replizierbares Plasmid eingebracht. Zur Verbesserung der Expression des *panBCD* Operons wurden starke, konstitutive Promotoren des *Bacillus subtilis* Phagen SP01 (P₂₆) verwendet und die Ribosomenbindungsstelle (=RBS) vor dem *panB*-Gen durch eine artifizielle RBS ersetzt. Vor die P₂₆*panBCD* Kassette auf dem Plasmid wurde ein DNA Fragment, das unmittelbar *upstream* des nativen *panB* Gens in *Bacillus* liegt, ligiert. Dieses Plasmid wurde in den *Bacillus subtilis* Stamm RL-1 (durch klassische Mutagenese erhaltenes Derivat des *Bacillus subtilis* 168 (Marburg Stamm ATCC 6051), Genotyp *trpC2* (Trp⁻)) transformiert und durch homologe Rekombination wurde das native *panBCD* Operon durch das p₂₆panBCD Operon ersetzt. Der resultierende Stamm heißt PA221 und hat den Genotyp P₂₆*panBCD, trpC2* (Trp⁻).
Mit dem *Bacillus subtilis* Stamm PA221 wurde in 10 ml Kulturen mit SVY-Medium, das mit 5 g/L β-Alanin und 5 g/L α-Ketoisovalerat supplementiert war, Pantothensäure-Titer von bis zu 0,92 g/L (24 h) erreicht.

Die Herstellung des *Bacillus subtilis* Stamms PA303 (Genotyp P₂₆*panE1*) ist in folgendem Abschnitt beschrieben :
Mit Hilfe der *E. coli panE* Gensequenz wurde die *Bacillus panE* Sequenz analog kloniert. Es zeigte sich, daß in *B. subtilis* zwei Homologe des *panE* Gens von *E. coli* existieren, die mit *panE1* und *panE2* bezeichnet wurden. Durch Deletionsanalysen zeigte sich, daß das *panE1* Gen für 90 % der Pantothensäure Produktion verantwortlich ist, während die Deletion des *panE2* Gens keinen signifikanten Effekt auf die Pantothensäure Produktion hatte. Auch hier wurde analog zur Klonierung des *panBCD* Operons der Promotor durch den starken konstitutiven Promotor P₂₆ ersetzt und die Ribbsomenbindungsstelle vor dem *panE1* Gen durch die artifizielle Bindungsstelle ersetzt. Das P₂₆*panE1* Fragment wurde in einen Vektor kloniert, des so gestaltet war, daß das P₂₆*panE1* Fragment in den original *panE1* locus im Genom von *Bacillus subtilis* integrieren konnte. Der nach Transformation und homologer Rekombination resultierende Stamm heißt PA303 und hat den Genotyp P₂₆*panE1.*
Mit dem *Bacillus subtilis* Stamm PA303, wurde in 10 ml Kulturen mit SVY-Medium, das mit 5 g/L β-Alanin und 5 g/L α-Ketoisovalerat supplementiert war, Pantothensäure-Titer von bis zu 1,66 g/L (24 h) erreicht.

Die weitere Stammkonstruktion erfolgte durch Transformation von PA327 mit einem Plasmid, welches das P₂₆*ilvBNC* Operon und das Marker-Gen für Spectinomycin enthielt. Das P₂₆*ilvBNC* Operon integrierte in den *amyE* locus, was durch PCR nachgewiesen wurde. Ein Transformante wurde als PA340 (Genotyp P₂₆*panBCD,* P₂₆*panE1,* P₂₆*ilvBNC,* specR, *trpC2* (Trp⁻)) bezeichnet.
Mit dem *Bacillus subtilis* Stamm PA340 wurde in 10 ml Kulturen mit SVY-Medium, das nur mit 5 g/L β-Alanin supplementiert war, Pantothensäure-Titer von bis zu 3,6 g/L (24 h) erreicht, in 10 ml Kulturen mit SVY-Medium, das mit 5 g/L β-Alanin und 5 g/L α-Ketoisovalerat supplementiert war, wurden Pantothensäure-Titer von bis zu 4,1 g/L (24 h) erreicht.

Des weiteren wurde in den Stamm PA340 eine deregulierte *ilvD*-Kassette eingebracht. Dazu wurde ein Plasmid, welches das *ilvD* Gen unter der Kontrolle des P₂₆ Promotors mit der artifiziellen RBS2 enthält, in PA340 transformiert. Dabei wurde das P₂₆*ilvD* Gen durch homologe Rekombination in den original *ilvD* locus integriert. Der resultierende Stamm PA374 hat den Genotyp P₂₆*panBCD,* P₂₆*panE1,* P₂₆*ilvBNC,* P₂₆*ilvD,* specR und *trpC2* (Trp⁻).
Mit dem *Bacillus subtilis* Stamm PA374 wurde in 10 ml Kulturen mit SVY-Medium, das nur mit 5 g/L β-Alanin supplementiert war, Pantothensäure-Titer von bis zu 2,99 g/L (24 h) erreicht.

Um mit dem Stamm PA374 β-Alanin zufütterungsfrei Pantothensäure zu produzieren, wurden zusätzliche Kopien des für die Aspartat-α-decarboxylase kodierenden Gens *panD* in den Stamm PA374 eingebracht. Dazu wurde chromosomale DNA des Stammes PA401 in PA374 transformiert. Durch Selektion auf Tetrazyklin wurde der Stamm PA377 erhalten.
Der resultierende Stamm PA377 hat den Genotyp P₂₆*panBCD,* P₂₆*panE1,* P₂₆*ilvBNC,* P₂₆*ilvD,* specR, tetR und *trpC2* (Trp⁻).

Mit dem *Bacillus subtilis* Stamm PA377 wurde in 10 ml Kulturen mit SVY-Medium Vorstufen-zufütterungsfrei Pantothensäure-Titer von bis zu 1,31 g/L (24 h) erreicht.

Die Herstellung des *Bacillus subtilis* Stamms PA401 (Genotyp P₂₆*panD*) ist in folgendem Abschnitt beschrieben :
Das Bacillus subtilis *panD* Gen wurde aus dem *panBCD* Operon in einen Vektor, der das Tetrazyklin Marker Gen trägt, kloniert. Vor das *panD* Gen wurde der Promotor P₂₆ und eine oben beschriebene artifizielle RBS kloniert. Durch Restriktionsverdau wurde ein Fragment, das das Tetrazyklin Marker Gen und das P₂₆*panD* Gen enthielt, hergestellt. Dieses Fragment wurde religiert und in den oben beschriebenen Stamm PA221 transformiert. Dabei integrierte das Fragment in das Genom des Stamms PA211. Der resultierende Stamm PA401 hat den Genotyp P₂₆*panBCD,* P₂₆*panD,* tetR und *trpC2* (Trp⁻).
Mit dem *Bacillus subtilis* Stamm PA401 wurde in 10 ml Kulturen in SVY-Medium, das mit 5 g/L α-Ketoisovalerat supplementiert war, Pantothensäure-Titer von bis zu 0,3 g/L (24 h) erreicht. In 10 ml Kulturen mit SVY-Medium das mit 5 g/L D-Pantoinsäure und 10 g/L L-Aspartat supplementiert war, wurden Pantothensäure-Titer von bis zu 2,2 g/L (24 h) erreicht.

Ausgehend von Stamm PA377 wurde durch Transformation mit chromosomaler DNA von Stamm PY79 ein Tryptophan prototropher Stamm generiert. Dieser Stamm PA824 hat den Genotyp P₂₆*panBCD,* P₂₆*panE1,* P₂₆*ilvBNC,* P₂₆*ilvD,* specR, tetR und Trp⁺.
Mit dem *Bacillus subtilis* Stamm PA824 wurde in 10 ml Kulturen in SVY-Medium Vorstufen-zufütterungsfrei Pantothensäure-Titer von bis zu 4,9 g/L (48 h) (Vergleich PA377: bis zu 3,6 g/L in 48 h) erreicht.

Die Herstellung von PA668 ist in folgendem Abschnitt beschrieben:
Das *Bacillus panB* Gen wurde aus dem Wild-Typ *panBCD* Operon kloniert und in einen Vektor insertiert, der neben einem Chloramphenicol-Resistenz-Gen auch *B. subtilis* Sequenzen des *vpr locus* enthält.
Der starke konstitutive Promotor P₂₆ wurde vor das 5' Ende des *panB* Gens eingeführt. Ein Fragment, das das P₂₆*panB* Gen, das Marker-Gen für Chloramphenicol-Resistenz sowie *Bacillus subtilis vpr* Sequenzen enthält wurde durch Restriktionsverdau erhalten. Das isolierte Fragment wurde religiert und der Stamm PA824 damit transformiert. Der erhaltene Stamm wurde mit PA668 bezeichnet. Der Genotyp von PA668 ist: P₂₆*panBCD,* P₂₆*panE1,* P₂₆*ilvBNC,* P₂₆*ilvD,* P₂₆*panB,* specR, tetR, CmR and Trp⁺.
Zwei Kolonien von PA668 wurde isoliert und mit PA668-2A, die andere mit PA668-24 bezeichnet.
Mit *B. subtilis* Stamm PA668-2A werden in 10mL Kulturen in SVY-Medium ohne Zufütterung von Vorstufen Pantothensäure-Titer von 1,5 g/L in 48 h erreicht. In 10 mL Kulturen, die mit 10g/L Aspartat supplementiert sind, werden Titer bis zu 5 g/L erreicht.
Mit *B. subtilis* Stamm PA668-24 werden in 10mL Kulturen in SVY-Medium ohne Zufütterung von Vorstufen Pantothensäure-Titer von 1,8 g/L in 48 h erreicht. In 10 mL Kulturen, die mit 10g/L L-Aspartat supplementiert sind, werden Titer bis zu 4,9 g/L erreicht.

Die genaue Konstruktion der Stämme ist gemäß den Anlagen der WO 01/21772 und US 2004/0091979 zu entnehmen.

Mit dem oben beschriebenen Stamm PA377 wird bei Glucose-limitierter Fermentation in SVY-Medium (25 g/L Difco Veal Infusion Broth, 5 g/L Difco Yeast Extract, 5 g/L Tryptophan, 5 g/L Na-Glutamat, 2 g/L (NH₄)₂SO₄, 10 g/L KH₂PO₄, 20 g/L K₂HPO₄, 0,1 g/L CaCl₂, 1 g/L MgSO₄, 1 g/L Natriumcitrat, 0,01 g/L FeSO₄x7 H₂O und 1 ml/L einer Spurensalzlösung folgender Zusammensetzung : 0,15 g Na₂MoO₄ x 2 H₂O, 2,5 g H₃BO₃, 0,7 g CoCl₂ x 6 H₂O, 0,25 g CuSO₄ x 5 H₂O, 1,6 g MnCl₂ x 4 H₂O, 0,3 g ZnSO₄ x 7 H₂O, mit Wasser auf 1 I aufgefüllt)) im 10 L Maßstab bei kontinuierlicher Zufütterung einer Glucose-Lösung in 36 h (48 h) Pantothensäure-Konzentrationen in der Fermentationsbrühe von 18-19 g/L (22-25 g/L) erreicht.

Bei Glucose-limitierter Fermentation von PA824, dem Tryptophan-prototrophen Derivat von PA377, in Hefeextrakt-Medium (10 g/L Difco Yeast Extract, 5 g/L Na-Glutamat, 8 g/L (NH₄)₂SO₄, 10 g/L KH₂PO₄, 20 g/L K₂HPO₄, 0,1 g/L CaCl₂, 1 g/L MgS0₄, 1 g/L Natriumcitrat, 0,01 g/L FeSO₄x7 H₂O und 1 ml/L der oben beschriebenen Spurensalzlösung) werden im 10 L Maßstab bei kontinuierlicher Zufütterung einer Glucose-Lösung in 36 h, 48 h und 72 h folgende Pantothensäure-Konzentrationen in Fermentationsbrühen erreicht: 20 g/L, 28 g/L und 36 g/L.

Durch weitere Medienoptimierung wird mit Stamm PA824 bei Glucose-limitierter Fermentation in einem Medium bestehend aus 10 g/L Difco Yeast Extract, 10 g/L NZ Amine A (Quest International GmbH, Erftstadt), 10 g/L Na-Glutamat, 4 g/L (NH₄)₂SO₄, 10 g/L KH₂PO₄, 20 g/L K₂HPO₄, 0,1 g/L CaCl₂, 1 g/L MgSO₄, 1 g/L Natriumcitrat, 0,01 g/L FeSO₄x7 H₂O und 1 ml/L der oben beschriebenen Spurensalzlösung im 10 L Maßstab bei kontinuierlicher Zufütterung einer Glucose-Lösung in 36h (48h) Pantothensäure-Konzentrationen von 37 g/L (48 g/L) in Fermentationsbrühen erreicht.
Weitere Erhöhungen der Pantothensäure-Konzentration in der Fermentationsbrühe sind durch weitere Medienoptimierung, durch Erhöhung der Fermentationsdauer, durch Verfahrens- und Stammverbesserung sowie durch Kombinationen der einzelnen Schritte denkbar. So sind die oben beschriebenen Pantothensäure-Konzentrationen auch durch Fermentation von Stämmen zu erreichen, die Derivate des oben beschriebenen PA824 sind. Derivate können durch klassische Stammentwicklung sowie durch weitere gentechnische Manipulationen hergestellt werden. Durch Medien-, Stamm- und Fermentationsverfahrensentwicklung können die Pantothensäure-Titer in den Fermentationsbrühen auf über 40, 45, 50, 55, 60, 65, 70, 75, 80, 85, und > 90 g/L gesteigert werden.

Ein wesentlicher Vorteil des erfindungsgemäßen Verfahrens ist, daß die Fermentation in einem Kulturmedium durchgeführt wird, das außer wenigstens einer Kohlenstoff- und Stickstoffquelle als Ausgangsverbindungen keine weiteren Vorstufen (Precursor) enthält. D.h. die Biosynthese von D-Pantothensäure ist von der Zufütterung weiterer Vorstufen unabhängig. Unter solchen Vorstufen sind erfindungsgemäß Substanzen wie z.B. β-Alanin und/oder L-Aspartat und/oder L-Valin und/oder α-Ketoisovalerat und/oder deren Kombinationen zu verstehen.
In einer bevorzugten Variante des erfindungsgemäßen Verfahren wird die Fermentation des D-Pantothensäure produzierenden Mikroorganismus in einem Kulturmedium durchgeführt, welches eine Kohlenstoff- und eine Stickstoffquelle enthält, dem aber kein freies β-Alanin und/oder β-Alanin-Salze zugesetzt ist oder im Verlauf der Fermentation zugeführt wird. D. h. zur Herstellung von D-Pantothensäure in Bereichen von wenigstens 10 g/l Kulturmedium, bevorzugt von wenigstens 20 g/l, besonders bevorzugt von wenigstens 40 g/l, höchst besonders bevorzugt von wenigstens 60 g/l und insbesondere von wenigstens 70 g/l, ist erfindungsgemäß keine Zufütterung von freiem β-Alanin und/oder β-Alanin-Salzen erforderlich.
Die Unabhängigkeit von der Zufütterung von Vorstufen stellt insbesondere einen wesentlichen wirtschaftlichen Vorteil des erfindungsgemäßen Verfahrens gegenüber bekannten Verfahren dar, da eine Vielzahl von Vorstufen sehr teuer sind.

Die Zugabe von β-Alanin und/oder β-Alanin-Salzen ist erfindungsgemäß jedoch nicht ausgeschlossen, so daß folglich die Ausbeute an D-Pantothensäure durch die Zugabe von β-Alanin und/oder β-Alanin-Salzen noch weiter verbessert werden kann.
Geht man beispielsweise davon aus, daß alle erforderlichen Vorstufen der Pantothensäure in ausreichender Menge vorhanden sind lediglich die Aktivität des panD-Gens eine weitere Steigerung der Pantothensäure-Produktion limitiert, so kann die Ausbeute an Pantothensäure z.B. um weitere 50% durch die Zugabe von freiem β-Alanin und/oder β-Alanin-Salzen gesteigert werden.
In einer vorteilhaften Variante der vorliegenden Erfindung können bis zu 20 g/l an freiem β-Alanin und/oder β-Alanin-Salzen dem Kulturmedium zur zusätzlichen Steigerung der Pantothensäureausbeute um mehr als 50 % zugesetzt werden. Bevorzugt ist der Zusatz von etwa 15 g/l an freiem β-Alanin und/oder β-Alanin-Salzen zum Kulturmedium.

Beispiele erfindungsgemäß geeigneter Kohlenstoffquellen zum Einsatz in einem Kulturmedium zur Fermentation der zuvor genannten Mikroorganismen sind Zucker, wie Stärkehydrolysate (Mono-, Di-, Oligosaccharide), bevorzugt Glucose oder Saccharose sowie Rüben- oder Rohrzuckermeiasse, Proteine, Proteinhydrolysate, Sojamehl, Maisquellwasser, Fette, freie Fettsäuren, rückgeführte Zellen aus bereits durchgeführten Fermentationen oder deren Hydrolysate sowie Hefeextrakt. Diese Aufzählungen sind nicht limitierend für die vorliegende Erfindung.

Ferner zeichnet sich das vorliegende Verfahren in vorteilhafter Weise dadurch aus, daß der Gesamt-Zuckergehalt bis zum Ende der Fermentation auf ein Minimum reduziert wird, da dieser anderenfalls die spätere Trocknung und/oder Formulierung der Fermentationslösung durch Verkleben erschwert. Dies kann erfindungsgemäß dadurch erreicht werden, daß die Fermentation noch einige Zeit weitergeführt wird, nachdem die Kohlenstoffquelle verbraucht ist (bei Kultivierung im batch-Betrieb) oder nachdem die Kohlenstoffzufuhr (bei einer Prozeßführung im fed-batch oder repeated-fed-batch-Betrieb) unterbrochen ist und/oder derart reguliert ist, daß die Konzentration der Kohlenstoffquelle nahezu Null ist (bei fed-batch, repeated-fed-batch oder kontinuierlicher Prozeßführung).
Dies erfolgt erfindungsgemäß dadurch, daß nach Unterbrechung der Zudosierung der Kohlenstoffquelle (z. B. Zuckerlösung) die Fermentation bis zum Erreichen der Gelöstsauerstoffkonzentration (pO₂) auf wenigstens 80 %, bevorzugt 90 % und besonders bevorzugt 95 % des Sättigungswertes in der Fermentationslösung weitergeführt wird.

Beispiele erfindungsgemäß geeigneter Stickstoffquellen sind Ammoniak, Ammoniumsulfat, Harnstoff, Proteine, Proteinhydrolysate oder Hefeextrakt. Auch diese Aufzählung ist nicht limitierend für die vorliegende Erfindung.

Ferner enthält das Fermentationsmedium Mineralsalze und/oder Spurenelemente, wie Aminosäuren und Vitamine. Die genauen Zusammensetzungen geeigneter Fermentationsmedien sind zahlreich bekannt und dem Fachmann zugänglich.
Nach Inockulation des Fermentationsmediums mit einem geeigneten D-Pantothensäure produzierenden Mikroorganismus (mit dem Fachmann bekannten Zelldichten) erfolgt, ggf. unter Zusatz eines Antischaummittels, die Kultivierung des Mikroorganismus. Eine ggf. erforderliche Regulierung des pH-Wertes des Mediums kann mit verschiedenen anorganischen oder organischen Laugen oder Säuren erfolgen, wie z.B. NaOH, KOH, Ammoniak, Phosphorsäure, Schwefelsäure, Salzsäure, Ameisensäure, Bersteinsäure, Zitronensäure o.ä..

Aufgrund der während der Fermentation eingesetzten Puffersysteme, die wie zuvor beschrieben, z.B. NaOH, KOH, Ammoniak, Phosphorsäure, Schwefelsäure, Salzsäure, Ameisensäure, Bersteinsäure, Zitronensäure o.ä. sein können, liegt die gebildete D-Pantothensäure in der Fermentationslösung je nach eingesetztem Puffersystem in Form des/der jeweiligen Salze(s) vor. Da hierbei erfindungsgemäß vor allem die Salze der D-Pantothensäure in Form ihrer einwertigen Kationen unvorteilhaft sind bzw. D-Pantothensäure in Form ihrer Calcium- oder Magnesiumsalze bevorzugt sind, wird die Fermentationslösung erfindungsgemäß über Elektromembrantrennverfahren aufbereitet.

Die vorliegende Erfindung umfaßt hierbei alle verfügbaren Arten von Elektromembrantrennverfahren, wie Membranelektrolysen oder Elektrodialysen. Es gehört zum Wissen des Fachmannes eine geeignete Auswahl zu treffen.
Erfindungsgemäß bevorzugt ist der Einsatz der Elektrodialyse. Hierbei kommen sowohl Elektrodialysen mit ausschließlich monopolaren Membranen als auch Elektrodialysen mit mono- und/oder bipolaren Membranen zum Einsatz. Besonders bevorzugt sind Elektrodialysen mit ausschließlich monopolaren Membranen, insbesondere Elektrodialysen mit für einwertige Ionen selektiven monopolaren Membranen, sogenannte monoselektive Membranen.
Es können prinzipiell beliebige, üblicherweise im Rahmen von Elektrodialyseverfahren eingesetzte Membranen zur Durchführung des erfindungsgemäßen Verfahren benutzt werden. Bei der im Rahmen der erfindungsgemäßen Elektrodialyse eingesetzten Membran werden vorzugsweise handelsübliche Ionentauschermembranen eingesetzt.
Als verwendbare Anionentauschermembranen sind beispielsweise zu nennen: Neosepta AM1, AM2, AM3, AMX, AMH, AFN, von Tokuyama Corp. und AMV von Asahi Glass.
Als Kationentauschermembranen sind Neosepta CM1, CM2, CMX, CMH, CMB, Asahi Glass CMV sowie Nafion 435 oder 350 von Du Pont de Nemours beispielhaft zu nennen.
Als monoselektive Kationenaustauschermembran ist z.B. die Membran Neosepta CMS zu nennen.
Als monoselektive Anionenaustauschermembran kommen beispielsweise die Membranen Neosepta ACS von Tokuyama Corp. Oder Selmion ASV von Asahi Glass in Betracht.
Als Elektroden könne Edelstähle, Nickel, Edelmetalle wie z.B. Platin oder andere dem Fachmann bekannte, geeignete Materialien eingesetzt werden.

Durch den Einsatz der monopolaren Elektrodialyse können erfindungsgemäß sowohl Anionen wie auch Kationen aus der Pantothenat-haltigen Lösung durch Migration im elektrischen Feld durch Anionen- bzw. Kationentauschermembranen in eine zweite Lösung (Konzentratlösung, KONZ) überführt und somit aus der Pantothensäure-haltigen Lösung (Diluatlösung, DIL) entfernt werden. Vorteilhaft werden erfindungsgemäß niedermolekulare Ionen aus der Pantothensäure-haltigen Lösung entfernt. Unter niedermolekularen Ionen sind erfindungsgemäß solche zu verstehen, die durch die Zusammensetzung des Kulturmediums und/oder Puffersystems in die Fermentationsbrühe eingetragen werden, jedoch im Endprodukt unerwünscht sind. Beispielsweise handelt es sich dabei um folgende Ionen: Ammonium-, Kalium-, Natrium-, Eisen-, Chlorid-, Phospat- oder Sulfat-Ionen.

In einer weiteren Ausführungsvariante der vorliegenden Erfindung ist es möglich, einen Ionentausch von einwertigen Kationen, wie Na⁺- oder Ammonium-Ionen, gegen mehrwertige Kationen, wie Ca²⁺, durchzuführen. Hierzu werden für monovalente Ionen selektive Kationentauschermembranen eingesetzt. So wird z. B. in einem Kreislauf I eine Lösung von CaCl₂ vorgelegt und in einem zweiten, durch eine Kationentauschermembran von Kreislauf I getrennten Kreislauf II die einwertigen Kationen enthaltende Pantothensäurelösung vorgelegt. In eine dritte Kammer III hinein werden dann durch eine zwischen Kammer II und Kammer III liegende, für monovalente Ionen selektive Kationentauschermembran, bevorzugt monovalente Kationen überführt. Gleichzeitig werden durch eine zwischen Kammer I und Kammer III liegende Anionentauschermembran Anionen, wie Chlorid-Ionen, aus Kammer I in Kammer III überführt. Ca²⁺-Ionen werden aus Kammer I in Kammer II durch eine Kationentauschermembran überführt. Auf diese Weise wird direkt Calcium-D-Pantothenat erhalten. Beim Vorliegen eines Salzes einer schwachen Säure, wie z.B. Pantothensäure, läßt sich in einer solchen Anordnung auch freie D-Pantothensäure gewinnen, wenn anstelle von CaCl₂ eine wässrige HCl-Lösung in Kammer I vorgelegt wird.

Erfindungsgemäß umfaßt ist auch der Einsatz von Calcium- und/oder Magnesium-Salzen in Form anorganischer oder organischer Anionen. Vorteilhaft werden erfindungsgemäß Calcium- und/oder Magnesium-Chlorid, -Nitrat, -Hydroxid, -Formiat, -Acetat, -Propionat, -Glycinat oder -Lactat verwendet.

In einer Ausführungsvariante der vorliegenden Erfindung können bei Einsatz der bipolaren Elektrodialyse in vorteilhafter Weise Kationen bei gleichzeitiger Bereitstellung von Protonen - durch Wasserdissoziation in der bipolaren Membran - aus der Pantothenat-haltigen Lösung durch Kationentauschermembranen in eine zweite Lösung überführt und somit aus der Pantothensäure-haltigen Lösung entfernt werden. In der zweiten Lösung werden mit durch Wasserdissoziation in der bipolaren Membran bereitgestellten Hydroxidionen die korrespondierenden Basen der überführten Kationen gebildet.

In einer anderen Ausführungsform der vorliegenden Erfindung können bei Einsatz der bipolaren Elektrodialyse in vorteilhafter Weise Anionen bei gleichzeitiger Bereitstellung von Hydroxidionen - durch Wasserdissoziation in der bipolaren Membran - aus der Pantothenat-haltigen Lösung durch Anionentauschermembran in eine zweite Lösung überführt und somit aus der Pantothensäure-haltigen Lösung entfernt werden. In der zweiten Lösung werden mit durch Wasserdissoziation in der bipolaren Membran bereitgestellten Protonen die korrespondierenden Säuren der überführten Anionen gebildet.

Erfindungsgemäß umfaßt ist auch eine weitere Ausführungsvariante bei der durch Einsatz der bipolaren Elektrodialyse einerseits vorteilhaft Anionen bei gleichzeitiger Bereitstellung von Hydroxidionen - durch Wasserdissoziation in der bipolaren Membran - aus der Pantothenat-haltigen Lösung durch Anionentauschermembranen in einer zweite Lösung überführt und somit aus der Pantothensäure-haltigen Lösung entfernt werden und andererseits Kationen bei gleichzeitiger Bereitstellung von Protonen - durch Wasserdissoziation in der bipolaren Membran - aus der Pantothenat-haltigen Lösung durch Kationentauschermembranen in einer dritte Lösung überführt und somit aus der Pantothensäure-haltigen Lösung entfernt werden. In der zweiten Lösung werden mit den durch Wasserdissoziation in der bipolaren Membran bereitgestellten Protonen die korrespondierenden Säuren der überführten Anionen und in der dritten Lösung werden mit durch Wasserdissoziation in der bipolaren Membran bereitgestellten Hydroxidionen die korrespondierenden Basen der überführten Kationen gebildet.

Erfindungsgemäß bevorzugt bei dem vorliegenden Verfahren ist ferner die Einstellung des pH-Wertes der Pantothenat-haltigen Lösung auf den isoelektrischen Punkt von Pantothensäure mit Hilfe von Säuren oder Basen. Aufgrund dessen kann die Ausbeute an freier D-Pantothensäure weiter gesteigert bzw. der Verlust weiter minimiert werden.

Die monopolare Elektrodialyse ist eine besonders bevorzugte Variante, da sie sehr kostengünstig ist im Vergleich zu der bipolaren Elektrodialyse.

Die bipolare Elektrodialyse bietet sich insbesondere dann an, wenn Säuren oder Basen zur Einstellung des pH-Wert nicht zugegeben werden sollen oder die entstehende Säure oder Lauge weiterverwendet werden kann.

Alle Elektrodialysevarianten bieten erfindungsgemäß den Vorteil, daß keine Abwasser aus der Regenerierung von Ionentauscherharzen anfallen.

Durch den erfindungsgemäßen Einsatz der Elektrotrennverfahren werden bevorzugt die unerwünschten niedermolekularen Ionen (Fremdionen), wie Ammonium-, Kalium-, Natrium-, Eisen-, Chlorid-, Phospat- oder Sulfat-Ionen, aus der D-Pantothenat-haltigen Lösung entfernt. D.h. es entsteht(en) bevorzugt freie D-Pantothensäure oder die gewünschten Salze, wie Calcium- und/oder Magnesium-D-Pantothenat. Erfindungsgemäß wird der Gehalt an einwertigen Kationen, bevorzugt Ammonium-, Kalium- und/oder Natrium-Ionen, auf eine Konzentration von ≤ 1g/kg Lösung reduziert.

Auch der Gehalt an unerwünschten Anionen, wie z.B. Chlorid-, Sulfat- oder Phosphat-Ionen, kann durch die Wahl der Ionentauschermembran, bevorzugt durch den Einsatz einer Anionentauschermembran, wesentlich reduziert werden.

Die so erhaltene Lösung enthaltend freie D-Pantothensäure wird erfindungsgemäß durch die Zugabe an Calcium- und/oder Magnesiumbase auf einen pH-Wert von 3-10 eingestellt. Vorteilhaft ist ein pH-Wert von 5-10. Bevorzugt ist die Einstellung des pH-Wertes auf einen Wert von 5-9, besonders bevorzugt von 6-9 und ganz besonders bevorzugt von 6-8. Auf diese Weise wird eine Lösung enthaltend Calcium- und/oder Magnesium-Pantothenat erhalten. Bevorzugt wird der Lösung zur Neutralisation Calciumhydroxid, Calciumcarbonat, Calciumoxid, Magnesiumhydroxid und/oder basisches Magnesiumcarbonat in Form eines Feststoffes und/oder als wässrige Suspension zugegeben.
Hierbei ist es erfindungsgemäß bevorzugt, wenn die Neutralisation der freien D-Pantothensäure enthaltenden Lösung mit Hilfe einer Calcium- und/oder Magnesiumbase in Form einer wässrigen Suspension erfolgt. Durch den Einsatz einer wässrigen Dispersion erfolgt die Neutralisation schneller und ohne größere pH-Schwankungen als dies bei Einsatz eines entsprechenden Feststoffes der Fall ist.

Erfindungsgemäß zeichnet sich das Verfahren dadurch aus, daß der Lösung in Schritt c) eine wässrige Suspension enthaltend 2-55 Gew.-%, bevorzugt 10-50 Gew.-% und besonders bevorzugt 20-40 Gew.-% an Calciumhydroxid zugegeben wird.
Erfindungsgemäß umfaßt ist ferner ein Verfahren, bei dem der Lösung in Schritt c) eine wässrige Suspension enthaltend 2-65 Gew.-%, bevorzugt 10-50 Gew.-% und besonders bevorzugt 20-40 Gew.-% an Calciumcarbonat zugegeben wird. In einer weiteren Ausführungsvariante der vorliegenden Erfindung wird der Lösung in Schritt c) des erfindungsgemäßen Verfahrens eine wässrige Suspension enthaltend 2-60 Gew.-%, bevorzugt 10-50 Gew.-% und besonders bevorzugt 20-40 Gew.-% an Magnesiumhydroxid zugegeben. Erfindungsgemäß umfaßt ist auch ein Verfahren, wobei der Lösung in Schritt c) eine wässrige Suspension enthaltend 2-25 Gew.-%, bevorzugt 10-20 Gew.-% an basischem Magnesiumcarbonat zugegeben wird.

Die Trocknung und/oder Formulierung der Fermentationslösung erfolgt nach an sich bekannten Verfahren, wie beispielsweise Sprühtrocknung, Sprühgranulation, Wirbelschichttrocknung, Wirbelschichtgranulation, Trommeltrocknung oder Spin-flash Trocknung (Ullmann's Encyclopedia of Industrial Chemistry, 6^{th} edition, 1999, electronic release, Kapitel "Drying of Solid Materials"). Die Gaseintrittstemperatur bei Konvektionstrocknung liegt im Bereich von 100 - 280 °C, bevorzugt bei 120 - 210 °C. Die Gasaustrittstemperatur liegt bei 50 - 180 °C, bevorzugt bei 60 - 150 °C. Zur Einstellung einer gewünschten Partikelgrößenverteilung und der damit verbundenen Produkteigenschaften können Feinpartikel abgetrennt und zurückgeführt werden. Ferner kann Grobgut in einer Mühle gemahlen und ebenfalls anschließend zurückgeführt werden.

Erfindungsgemäß vorteilhaft ist bei dem zuvor dargestellten Verfahren die Reduzierung aufwendiger Aufarbeitungsschritte, insbesondere der Verzicht auf den Einsatz organischer Lösungsmittel, bei gleichzeitiger Bereitstellung eines gewünschten Produktes mit guter biologischer Wertigkeit. Ferner wird erfindungsgemäß die Menge an anfallendem Abwasser wesentlich reduziert. Dies resultiert somit in weiteren Einsparungen an aufwendigen Aufbereitungs- und Entsorgungsanlagen. Somit zeichnet sich das erfindungsgemäße Verfahren in vorteilhafter Weise dadurch aus, daß es einfacher, weniger störanfällig, weniger zeitaufwendig, deutlich kostengünstiger und damit wirtschaftlicher ist, als herkömmliche Verfahren.

Dies schließt jedoch nicht aus, daß das erfindungsgemäße Verfahren variiert werden kann. Die eingangs genannten erfindungsgemäßen Verfahrensschritte a) bis d) können durch einen oder mehrere der folgenden Verfahrensschritte ergänzt werden, die für sich genommen dem Fachmenschen vertraut sind. Hierbei sind alle denkbaren Kombinationen der zusätzlichen (fakultativen) Verfahrensschritte mit den (essentiellen) Verfahrensschritten a) bis d) erfindungsgemäß umfaßt.

So können die Lösungen resultierend aus den Verfahrensschritten a) - c) z. B. durch Erhitzen (Sterilisation) oder andere Methoden, wie z.B. Pasteurisierung oder Sterilfiltration entkeimt werden.

In weiteren Varianten des erfindungsgemäßen Verfahrens kann vor der Trocknung und/oder Formulierung der Lösung wenigstens einer und/oder Kombinationen der nachfolgenden Schritte durchgeführt werden, umfassend Lyse und/oder Abtötung der Biomasse und/oder Abtrennung der Biomasse von der Fermentationslösung und/oder Zugabe weiterer Zuschlagstoffe und/oder Konzentrierung der Fermentationslösung, bevorzugt durch Wasserentzug.

Gegenstand der vorliegenden Erfindung ist somit auch ein Verfahren, wobei die Lyse und/oder Abtötung der Biomasse noch in der Fermentationslösung oder erst nach der Abtrennung der Biomasse von der Fermentationslösung durchgeführt wird. Dies kann beispielsweise durch eine Temperaturbehandlung, bevorzugt bei 80 - 200 °C und/oder eine Säurebehandlung, bevorzugt mit Schwefelsäure oder Salzsäure und/oder enzymatisch, bevorzugt mit Lysozym erfolgen.

In einer weiteren Ausführungsform der vorliegenden Erfindung können die Zellen der fermentierten Mikroorganismen durch Filtration, Separation (z. B. Zentrifugieren) und/oder Dekantieren aus den Lösungen der Schritte a), b) oder c) des erfindungsgemäßen Verfahrens entfernt werden. Denkbar ist auch, daß die Lösungen der Schritte a), b) oder c) ohne die Abtrennung der enthaltenen Mikroorganismen direkt durch Anlegen eins elektrischen Feldes über eine oder mehrere ionenselektive Membran(en) geleitet werden.

Die aus der Aufarbeitung über Membranelektrolyse oder Elektrodialyse resultierende Lösung kann im Anschluß an die Neutralisation über einen geeigneten Verdampfer, z. B. Fallfilmverdampfer, Dünnschichtverdampfer oder Rotationsverdampfer aufkonzentriert werden. Solche Verdampfer werden z. B. von den Firmen GIG (4800 Attnang Puchheim, Österreich), GEA Canzler (52303 Düren, Deutschland), Diessel (31103 Hildesheim, Deutschland) und Pitton (35274 Kirchhain, Deutschland) hergestellt.

Um die farblichen Eigenschaften des Endproduktes zu verbessern, kann ein zusätzlicher Filtrationsschritt durchgeführt werden, bei dem den während des Verfahrens erhaltenen Lösungen etwas Aktivkohle zugesetzt wird und diese Suspension anschließend filtriert wird. Oder die während der Fermentation erhaltenen Lösungen können über ein kleines Aktivkohlebett geleitet werden. Die hierzu erforderlichen Mengen eingesetzter Aktivkohle liegen im Bereich weniger Gew.-% der Lösung und liegen im Wissen und Ermessen des Fachmanns.
Diese Filtrationen können erleichtert werden, indem der jeweiligen Lösung vor der Filtration ein handelsübliches Flockungshilfsmittel (z. B. Sedipur CF 902 oder Sedipur CL 930 der Firma BASF AG, Ludwigshafen) zusetzt wird.

In einer vorteilhaften Ausführungsform der vorliegenden Erfindung wird der Fermentationsaustrag (Fermentationsbrühe) durch Erhitzen sterilisiert und dann durch Zentrifugieren, Filtrieren oder Dekantieren von der Zellmasse befreit. Nach Zusatz von 50 - 1000 mg/kg, bevorzugt 100- 200 mg/kg eins handelsüblichen Flockungshilfsmittels bezogen auf den Fermentationsaustrag wird über ein kurzes Bett von Aktivkohle und Sand filtriert, um eine Biomasse-freie Lösung mit hohem D-Pantothensäure-Gehalt zu erhalten. Anschließend wird diese aufbereitete Lösung durch Anlegen eins elektrischen Feldes über eine oder mehrere ionenselektive Membran(en) gefördert.

Die sich anschließende Trocknung dieser Lösung kann beispielsweise durch Sprühtrocknung erfolgen. Diese kann im Gleichstrom, Gegenstrom oder Mischstrom erfolgen. Zur Zerstäubung können alle bekannten Zerstäuber herangezogen werden, insbesondere Zentrifugalzerstäuber (Zerstäuberscheibe), Einstoffdüse oder Zweistoffdüse. Bevorzugte Trocknungstemperaturbedingungen sind 150 - 250 °C Turmeintrittstemperatur und 70-130 °C Turmaustrittstemperatur. Es kann aber auch bei höherem oder niedrigeren Temperatumiveau getrocknet werden. Um eine sehr niedrige Restfeuchte zu erzielen, kann noch ein weiterer Trocknungsschritt in einem Wirbelbett nachgeschaltet werden.

Die Sprühtrocknung läßt sich auch in einem FSD- oder SBD-Trockner (FSD: Fluidized Spray Dryer; SBD: Spray Bed Dryer), wie sie von den Firmen Niro (Kopenhagen, Dänemark) und APV-Anhydro (Kopenhagen, Dänemark) gebaut werden, durchführen, die eine Kombination von Sprühtrockner und Wirbelbett darstellen.
Bei der Sprühtrocknung kann ein Fließhilfsmittel zugesetzt werden. Dadurch können die Beläge an der Trocknerwandung reduziert und das Fließverhalten, gerade bei feinkörnigen Pulvern verbessert werden. Als Fließhilfsmittel kommen insbesondere Silikate, Stearate, Phosphate und Maisstärke in Betracht.
Prinzipiell kann die Trocknung auch in einer Sprühwirbelschicht erfolgen, wobei diese sowohl kontinuierlich als auch diskontinuierlich betrieben werden kann. Das Einsprühen der Lösung kann sowohl von oben (Topspray), von unten (Bottomspray) als auch von der Seite (Sidespray) erfolgen.

Gegenstand der vorliegenden Erfindung ist ferner eine Zusammensetzung für den Einsatz als Tierfutterzusatz und/oder Tierfutterergänzungsmittel, wobei sie herstellbar ist, indem
a) wenigstens ein D-Pantothensäure produzierender Mikroorganismus eingesetzt wird, dessen Pantothensäure-(pan)- und/oder Isoleucin/Valin-(ilv)-Biosynthese dereguliert ist und der wenigstens 2 g/l an Salzen der D-Pantothensäure durch Fermentation in einem Kulturmedium bildet, wobei dem Kulturmedium 0 - 20 g/l, bevorzugt 0 g/l freies β-Alanin und/oder β-Alanin-Salz zugeführt wird,
b) die D-Pantothenat enthaltende Fermentationslösung durch Anlegen eins elektrischen Feldes über eine oder mehrere ionenselektive Membran(en) geleitet wird, wobei niedermolekulare Ionen aus der D-Pantothenat-haltigen Lösung entfernt werden,
c) die in der Lösung enthaltene freie D-Pantothensäure durch die Zugabe einer Calcium- und/oder Magnesiumbase auf einen pH-Wert von 3-10 eingestellt wird, wobei eine Lösung erhalten wird, die Calcium- und/oder Magnesium-Pantothenat enthält und
d) die Calcium- und/oder Magnesium-Pantothenat enthaltende Lösung einer Trocknung und/oder Formulierung unterzogen wird.

In einer Variante der vorliegenden Erfindung zeichnet sich die Zusammensetzung dadurch aus, daß in Schritt c) oder d) eine Suspension erhalten oder vorgelegt wird, die Calcium- und/oder Magnesium-Pantothenat enthält.

Erfindungsgemäß zeichnet sich die Zusammensetzung ferner dadurch aus, daß sie Salze der D-Pantothensäure in einer Konzentration von wenigstens 1-100 Gew.-%, bevorzugt wenigstens 20-100 Gew.-% und besonders bevorzugt wenigstens 50 Gew.-% enthält. Gegenstand der vorliegenden Erfindung ist eine Zusammensetzung, die Salze der D-Pantothensäure in Form zweiwertiger Kationen, bevorzugt Calcium-und/oder Magnesium-D-Pantothenat enthält. Erfindungsgemäß bevorzugt ist eine Zusammensetzung, die sich dadurch auszeichnet, daß der Gehalt an Salzen der D-Pantothensäure in Form einwertiger Kationen ≤ 1 g/kg ist.

Erfindungsgemäß wird durch das zuvor beschriebene Verfahren ein Calcium- oder Magnesium-D-Pantothenat erhalten, das den Ansprüchen für einen Futtermittelzusatzstoff genügt. Diese Anforderungen sind beispielsweise ein relativ hoher Gehalt an D-Pantotheriat und eine gute Verträglichkeit für den Zielorganismus sowie eine biologische Wertigkeit im Sinne der "Vitamin-Wirkung" des erfindungsgemäßen Produktes.

Die vorliegende Erfindung wird durch die nachfolgenden Beispiele näher erläutert, die jedoch nicht limitierend für die Erfindung sind:

### Beispiel 1:

In einem Laborfermenter mit Rührer und Begasungseinrichtung mit 14 I Inhalt wurde wässriges Fermentationsmedium mit der folgenden Zusammensetzung vorgelegt :

| Einsatzstoff | Konzentration [g/l] |
|---|---|
| Hefeextrakt | 10 |
| Natriumglutamat | 5 |
| Ammoniumsulfat | 8 |
| Antischaum | 1 ml/l |

Nach der Sterilisation wurden folgende sterile Medienkomponenten zusätzlich zugegeben:

| Einsatzstoff | Konzentration [g/l] |
|---|---|
| KH₂PO₄ | 10 |
| K₂HPO₄ | 20 |
| Glucose | 2,5 |
| Calciumchlorid | 0,1 |
| Magnesiumchlorid | 1 |
| Natriumcitrat | 0,1 |
| FeSO₄ x 7 H₂O | 0,1 |
| Spurensalzlösung | 1 ml/l |

Die Spurensalzlösung setzt sich wie folgt zusammen :
0,15 g Na₂MoO₄ x 2 H₂O, 2,5 g H₃BO₃, 0,7 g CoCl₂ x 6 H₂O, 0,25 g CuSO₄ x 5 H₂O, 1,6 g MnCl₂ x 4 H₂O, 0,3 g ZnSO₄ x 7 H₂O werden mit Wasser auf 1 l aufgefüllt. Die Zugabe der Spurensalzlösung erfolgt über eine Sterilfiltration. Das Anfangsflüssigkeitsvolumen beträgt 8,4 l. Die oben angeführten Gehalte sind auf diesen Wert bezogen.

Dieser Lösung wird 160 ml Impfkultur (OD = 10 in SVY-Medium (SVY-Medium: Difco Veal Infusion broth 25 g, Difco Yeast extract 5 g, Natriumglutamat 5 g, (NH₄)₂SO₄ 2.7 g in 740 ml H₂O, sterilisieren; 200 ml sterile 1 M K₂HPO₄ (pH 7) und 60 ml sterile 50% Glucose-Lösung zusetzen (Endvolumen 1L))) von *Bacillus subtilis* PA824 zugesetzt und bei 43°C unter starkem Rühren bei einer Begasungsrate von 12 l/min fermentiert. Dieser Stamm ist gemäß der Anlage in der WO 01/21772 beschrieben.

Innerhalb von 52 h wurden 6 l einer sterilen wässrigen Lösung zudosiert. Die Zusammensetzung war:

| Einsatzstoff | Konzentration [g/l] |
|---|---|
| Glucose | 500 |
| Calciumchlorid | 0,6 |
| Spurensalzlösung | 7 ml/l |

Die Fermentation wurde Glucose-limitiert durchgeführt. Während der Fermentation wurde der pH Wert bei 7,2 durch die Zudosierung von 25%iger Ammoniaklösung bzw. von 20 %igerPhosporsäure gehalten. Ammoniak dient gleichzeitig als Stickstoffquelle für die Fermentation. Die Drehzahl des Rührorgans wurde geregelt, indem der Gelöstsauerstoffgehalt auf 30% des Sättigungswertes gehalten wurde. Das Schäumen wurde durch gelegentliches Zudosieren eines Antischaummittels kontrolliert. Nach Abbruch der Zudosierung der Kohlenstoffquelle wurde die Fermentation so lange weitergeführt, bis der Gelöstsauerstoffgehalt (pO₂) einen Wert von 95% des Sättigungswerts erreicht hatte. Danach wurde die Fermentation beendet und der Mikroorganismus thermisch abgetötet. Dazu wurde die Fermentationslösung 60 min sterilisiert. Die Abtötung wurde durch Ausplattieren nachgewiesen. Anschließend erfolgt die Zellabtrennung durch Zentrifugation. Nach Zellabtrennung beträgt die Konzentration an D-Pantothenat nach 52 h 24,7 g/l.

In analoger Weise lassen sich auch Fermentationsbrühen erzeugen, die β-Alaninzufütterungsfrei Pantothensäure-Titer von über 20, 25, 30, 35, 40, 45, 50, 55, 60, 65, 70, 75, 80, 85, und >90 g/L aufweisen.

Ein Ferrnentationsaustrag, der wie oben beschrieben durch Erhitzen sterilisiert und durch Zentrifugieren von der Biomasse weitgehend befreit worden ist, wurde mit zusätzlicher D-Pantothensäure versetzt und der pH-Wert mit Schwefelsäure auf ungefähr 2 eingestellt. Die so erhaltene Lösung wurde über ein kleines Bett von Sand/Aktivkohle filtriert. Der Gehalt an D-Pantothensäure in dieser Lösung beträgt 67,7g/l.

900g dieses Filtrats wurden wie folgt elektrodialytisch behandelt: In einer Elektrodialysezelle (ausgestattet mit Anionen- und Kationentauschermembranen (Typen Neosepta AMX Sb bzw. CMX Sb von Tokuyama Corp.) mit einer effektiven Zellfläche von 1,85 dm² und fünf Kammern, wurde das Filtrat bei einer anfänglichen Stromdichte von 29 mA/cm² entsalzt. Die Stromdichte nahm im Versuchsverlauf nach Erreichen der vorgegebenen Grenzspannung von 20 V kontinuierlich ab. Die Temperatur lag zwischen 33 °C und 40 °C. Als Konzentrateinsatz wurde 0,5%-ige (w/w) NaCl-Lösung vorgelegt. Dieses Konzentrat (KONZ) wurde im Versuchsverlauf mit den aus dem Filtrat (Diluat, DIL) entfernten Fremdionen (z. B. Ammonium-, Chlorid-, Eisen-, Kalium-, Natrium- oder Phosphat-Ionen) angereichert. Im Elektrolytkreis wurde zur Spülung der Elektroden 5%-ige (w/w) Natriumsulfatlösung vorgelegt. Im Versuchsverlauf wurden bei verschiedenen ionischen Leitfähigkeitswerten der Diluatlösung Proben aus der Diluat enthaltenden Kammer entnommen und analysiert. Die Analyse ist in der nachfolgenden Tabelle dargestellt und ergab, daß mit fortschreitender Versuchsdauer die genannten Ionen aus dem Diluat entfernt wurden und die Leitfähigkeit des Diluats entsprechend abnimmt. Kleine, einwertige Ionen, wie z. B. Chlorid-Ionen, werden schneller entfernt, als voluminöse, mehrwertige Ionen, wie z. B. Phosphat-Ionen.

Die Ergebnisse der Diluat-Analyse sind der nachfolgenden Tabelle zu entnehmen.

| | PTS | Ionen (U-Labor -Analyse) | | | | | | | |
|---|---|---|---|---|---|---|---|---|---|
| | g/l | Ammonium | Cl | o-Phosphat | Ca | Fe | K | Mg | Na |
| Edukt (mS/cm) | 67,7 | 0,35-0,40% | 0,90-0,97% | 0,61-0,79% | 0,009% | <0,001% | 0,45% | 0,002% | - |
| 4831/00/196 DIL 25 mS/cm | 67,9 | 0,22% | 0,65% | 0,56% | 0.005% | <0,001% | 0,30% | 0,002% | 0,089% |
| 4831/00/196 DIL 20 mS/cm | 70,7 | 0,16% | 0,49% | 0,53% | 0,004% | <0,001% | 0.23% | 0,002% | 0,077% |
| 4831/00/196 DIL 15 mS/cm | 64,1 | 0,12% | 0,32% | 0,54% | 0,003% | <0,001% | 0,16% | 0,001% | 0,063% |
| 4831/00/196 DIL 10 mS/cm | 73.2 | 0,08% | 0,16% | 0,48% | 0,002% | <0,001% | 0,099% | 0,001% | 0,047% |
| 4831/00/196 DIL 5 mS/cm | 79,9 | 0,03% | 0,04% | 0,35% | 0,001% | <0,001% | 0,038% | 0,001% | 0,025% |
| 48311001196 DIL 3 mS/cm | 75,6 | 0,02% | 0.01% | 0,26% | 0,001% | <0,001% | 0,016% | <0,001% | 0,015% |
| 4831/00/196 DIL-Ende | 76,0 | 0,01% | <0,01% | 0,21% | <0,001% | <0,001% | 0,009% | <0,001% | 0.010% |

| | | | | | | | | | |
|---|---|---|---|---|---|---|---|---|---|
| PTS = Pantothensäure | | | | | | | | | |

Die pH-Werte aller Diluat-Proben lag zwischen 2-3. In 771 g des Diluat-Austrags sind 76 g/l freie D-Pantothensäure enthalten.
Hiermit ist gezeigt, daß eine fermentativ erhaltene D-Pantothensäurelösung elektrolytisch erfolgreich von störenden Kationen befreit werden kann. Aus der erhaltenen entsalzten D-Pantothensäure läßt sich ein nicht-hygroskopisches Calcium-D-Pantothenat-Pulver, wie in Beispiel 2 beschrieben, herstellen.

### Beispiel 2:

In einem Laborfermenter mit Rührer und Begasungseinrichtung mit 14 1 Inhalt wurde wässriges Fermentationsmedium mit der folgenden Zusammensetzung vorgelegt :

| Einsatzstoff | Konzentration [g/l] |
|---|---|
| Hefeextrakt | 10 |
| Natriumglutamat | 5 |
| Ammoniumsulfat | 8 |
| KH₂PO₄ | 8,4 |
| K₂HPO₄ | 15 |

Nach der Sterilisation wurden folgende sterile Medienkomponenten zusätzlich zugegeben:

| Einsatzstoff | Konzentration [g/l] |
|---|---|
| Glucose | 2,5 |
| Calciumchlorid | 0,1 |
| Magnesiumchlorid | 1 |
| Natriumcitrat | 1 |
| FeSO₄ x 7 H₂O | 0,01 |
| Spurensalzlösung | 1 ml |

Die Spurensalzlösung setzt sich wie folgt zusammen :
0,15 g Na₂MoO₄ x 2 H₂O, 2,5 g H₃BO₃, 0,7 g CoCl₂ x 6 H₂O, 0,25 g CuSO₄ x 5 H₂O, 1,6 g MnCl₂ x 4 H₂O, 0,3 g ZnSO₄ x 7 H₂O werden mit Wasser auf 1 l aufgefüllt. Die Zugabe der Spurensalzlösung erfolgt über eine Sterilfiltration. Das Anfangsflüssigkeitsvolumen beträgt 5,5 l. Die oben angeführten Gehalte sind auf diesen Wert bezogen.

Dieser Lösung wird 55 ml Impfkultur (OD = 10 in SVY-Medium (SVY-Medium: Difco Veal Infusion broth 25 g, Difco Yeast extract 5 g, Natriumglutamat 5 g, (NH₄)₂SO₄ 2.7 g in 740 ml H₂O lösen, sterilisieren; 200 ml sterile 1 M K₂HPO₄ (pH 7) und 60 ml sterile 50% Glucose-Lösung zusetzen (Endvolumen 1L))) von *Bacillus subtilis* PA824. zugesetzt und bei 43 °C unter starkem Rühren bei einer Begasungsrate von 12 l/min fermentiert. Dieser Stamm ist gemäß der Anlage in der WO 01/21772 beschrieben.

Innerhalb von 48 h wurden 6 l einer sterilen wässrigen Lösung zudosiert. Die Zusammensetzung war:

| Einsatzstoff | Konzentration [g/l] |
|---|---|
| Glucose | 550 |
| Calciumchlorid | 0,6 |
| Spurensalzlösung | 6 ml |

Die Fermentation wurde Glucose-limitiert durchgeführt. Während der Fermentation wurde der pH Wert bei 7,2 durch die Zudosierung von 25%iger Ammoniaklösung bzw. von 20 % iger Phosporsäure gehalten. Ammoniak dient gleichzeitig als Stickstoffquelle für die Fermentation. Die Drehzahl des Rührorgans wurde geregelt, indem der Gelöstsauerstoffgehalt auf 30% des Sättigungswertes gehalten wurde. Das Schäumen wurde durch gelegentliches Zudosieren eines Antischaummittels kontrolliert. Nach Abbruch der Zudosierung der Kohlenstoffquelle wurde die Fermentation so lange weitergeführt, bis der Gelöstsauerstoffgehalt (pO₂) einen Wert von 95% des Sättigungswerts erreicht hatte. Danach wurde die Fermentation beendet und der Mikroorganismus thermisch abgetötet. Dazu wurde die Fermentationslösung 30 min sterilisiert. Die Abtötung wurde durch Ausplattieren nachgewiesen. Die Zellabtrennung erfolgte durch Zentrifugation. Nach Zellabtrennung betrug die Konzentration an D-Pantothenat nach 48 h 24,1 g/l.
In analoger Weise lassen sich auch Fermentationsbrühen erzeugen, die β-Alaninzufütterungsfrei Pantothensäure-Titer von über 20, 25, 30, 35, 40, 45, 50, 55, 60, 65, 70, 75, 80, 85, und >90 g/L aufweisen.
2817ml des Fermenteraustrages wurden mit 1183 ml einer Pantothensäurelösung der Konzentration von 178,9 g/l versetzt und mit 25%-iger Ammoniumhydroxid-Lsg. neutralisiert (pH 6,5). Anschließend wird über Sand/Aktivkohle filtriert. Der Gehalt an D-Pantothensäure im Filtrat betrug 67g/l, hauptsächlich in der Form des Ammoniumsalzes.
Ungefähr 720g dieser Lösung wurden elektrodialytisch gemäß Beispiel 1 behandelt:

550 g des Diluat-Austrages (mit einer Dichte von 1,017 g/ml) wurde mit 3,5 g einer 40%-igen Calciumhydroxid-Suspension auf einen pH-Wert von 7,5 eingestellt. Erhalten wurde eine wässrige Calcium-D-Pantothenatlösung (551,03 g mit einem Calcium-D-Pantothenatgehalt von 40,9 g/l).
Die wässrige Calciumpantothenat-Lösung wurde in einem Laborsprühtrockner der Fa. Niro, Typ Minor, getrocknet. Die Turmeintrittstemperatur betrug etwa 200°C, die Turmaustrittstemperatur 90°C. Die Zerstäubung erfolgte mittels Zweistoffdüse bei einem Druck von 2 bar. Als Puderungsmittel wurde Sipemat 22F zugesetzt. Dabei wurde ein pulvriges Produkt folgender Spezifikation erhalten (Angaben in Gew.-%):
Wassergehalt: 2,3%
Calcium-D-Pantothenat: 46,1 %
Ammonium: 0,60%
Kalium: 0,47%
Natrium: 1,1%

### Beispiel 3:

In einem Fermenter mit Rührer und Begasungseinrichtung mit 200 Liter Inhalt wurden 800 g 50%-iger Hefeextrakt, 438 g Na-Glutamat, 3200 g Sojamehl, 640 g Ammoniumsulfat, 800 g KH₂PO₄, 1600 g K₂HPO₄ und 50 ml Antischaummittel TegoKS mit 70 l Wasser versetzt und für 30min bei 121°C sterilisiert.

Anschließend wurden Lösung 1 und Lösung 2 zugegeben.

Lösung 1 wird wie folgt angesetzt: 1670 g Glucose x H₂O, 10,2 g CaCl₂ x 2 H₂O und 170,7 g MgCl₂ x 6 H₂O werden in 9 l Wasser gelöst und für 30 min sterilisiert.

Lösung 2 wird wie folgt angesetzt: 800 ml Citrat-Eisenlösung (200 g/l Natriumcitrat, 2 g/L FeSO₄ x 7 H2O, sterilfiltriert) werden mit 80 ml Spurensalzlösung (0,15 g Na₂MoO₄ x 2 H₂O, 2,5 g H₃BO₃, 0,7 g CoCl₂ x 6 H₂O, 0,25 g CuSO₄ x 5 H₂O, 1,6 g MnCl₂ x 4 H₂O, 0,3 g ZnSO₄ x 7 H₂O werden mit Wasser auf 1 l aufgefüllt, sterilfiltriert) versetzt.

Dieser Lösung wurde 2 l Impfkultur (OD = 10 in SVY-Medium (SVY-Medium: Difco Veal Infusion broth 25 g, Difco Yeast extract 5 g, Natriumglutamat 5 g, (NH₄)₂SO₄ 2.7 g in 740 ml H₂O, sterilisieren; 200 ml sterile 1 M K₂HPO₄ (pH 7) und 60 ml sterile 50% Glucose-Lösung zusetzen (Endvolumen 1 I))) von *Bacillus subtilis* PA668 zugesetzt und bei 43°C unter starkem Rühren bei einer Begasungsrate von 3 m³/min fermentiert. Dieser Stamm ist gemäß der Anlage in der US 2004/0091979 beschrieben.

Innerhalb von 48 h wurden ca. 10 l einer sterilen wässrigen Glucose-Lösung zudosiert. Die Lösung wurde wie folgt angesetzt: 90 kg Glucose x H₂O wurden mit 55 kg Wasser versetzt und für 30min sterilisiert. Anschließend wurden 300 ml Spurensalzlösung (Zusammensetzung s. oben) sowie 3 l Citrat-Eisenlösung (Zusammensetzung s. oben) zugesetzt. Anschließend wurde 11 sterilfiltrierter 90 g/l CaCl₂ x 2H₂O Lösung sowie 2 l sterilfiltrierte 375 g/l Natriumglutamat-Lösung zugegeben.

Die Fermentation wurde Glucose-limitiert durchgeführt. Während der Fermentation wurde der pH Wert bei 7,2 durch die Zudosierung von 25%iger Ammoniaklösung bzw. von 20%iger Phosporsäure gehalten. Ammoniak dient gleichzeitig als Stickstoffquelle für die Fermentation. Die Drehzahl des Rührorgans wurde geregelt, indem der Gelöstsauerstoffgehalt auf 30% des Sättigungswertes gehalten wurde. Das Schäumen wurde durch gelegentliches Zudosieren eines Antischaummittels kontrolliert. Nach Abbruch der Zudosierung der Kohlenstoffquelle wurde die Fermentation so lange weitergeführt, bis der Gelöstsaüerstoffgehalt (pO2) einen Wert von 95% des Sättigungswerts erreicht hatte. Danach wurde die Fermentation beendet. Die Zellabtrennung erfolgte durch Separation in einem Tellersparator. Restliche Zellen im Überstand wurden thermisch durch Sterilisation abgetötet. Die Konzentration an D-Pantothenat bei Abbruch nach 48 h betrug 13,7 g/l. Nach Separation und Sterilisation betrug die Konzentration an D-Pantothenat 10,7 g/l. In analoger Weise lassen sich auch Fermentationsbrühen erzeugen, die β-Alaninzufütterungsfrei Pantothensäure-Titer von mehr als 15, 20, 25, 30, 35, 40, 45, 50, 55, 60, 65, 70, 75, 80, 85, und mehr als 90 g/l aufweisen.

Mit 3000 g der so vorbehandelten Fermentationslösung wurde wie folgt weiter verfahren: zwei Drittel des Ansatzes wurden bei ca. 10°C mit einer der Äquivalentkonzentration an mehrwertigen Anionen entsprechenden Menge an CaCl₂ (40 % Lösung); ein Drittel des Ansatzes entsprechend mit einer äquivalenten Menge an Ca(OH)₂ (fest) gefällt. Die Lösungen wurden über Nacht im Kühlschrank gelagert und dann mittels einer Druckfiltemutsche (Seitz Tiefenfilter 700) vom ausgefällten Bodenkörper getrennt. Die Lösungen wurden danach gemischt und durch Zugabe von weiteren 10 g Ca(OH)₂ bei circa 10°C basisch gestellt (pH=8,7) und nachgefällt. Der nachgefällte Niederschlag wurde ebenfalls abfiltriert.

1800 g der Lösung wurden dann bei 35°C in einem Dreikreiselektrodialysestack umgesalzt. Dabei wurde die Fermentationsbrühe durch eine von zwei Kationentauschermembranen begrenzte Produktkammer geführt. Auf der Kathodenseite trennte eine monoselektive Kationenaustauschermembran (Tokuyama Soda, CMS) das Produkt vom Konzentratkreislauf, anodenseitig wurde die Produktkammer von einer herkömmlichen Kationenaustauschermembran (Tokuyama Soda, CMX) vom CaCl₂ Kreislauf getrennt. Konzentrat und CaCl₂ Kreislauf wurden durch eine Anionentauschermembran (Tokuyama Soda, AMX) getrennt. Im Konzentrat wurden 750 g einer 0,5 %-igen NaCI-Lösung, im CaCl₂ Kreis 900 g einer 3,05 %-igen CaCl₂ Lösung vorgelegt. Im Elektrodenspülkreislauf wurde eine 0,1 molare Na₂SO₄ Lösung zirkuliert. Der Versuch wurde galvanostatisch bei 30 mA/cm² in einem Stack bestehend aus fünf der oben beschriebenen Grundeinheiten mit einer Gesamtmembranfläche von 500 cm² im Batchbetrieb durchgeführt. Als Abbruchkriterium wurde eine minimale Leitfähigkeit von 4 mS/cm im CaCl₂ Kreis gewählt. Die Ergebnisse der Kationenanalytik in Produkt- und Konzentratkreislauf sind in der nachfolgenden Tabelle zusammengestellt.

| | PTS [g/l] | Ca²⁺ | Na⁺ | K⁺ | NH₄+ |
|---|---|---|---|---|---|
| 5329/01/82 Produkt t=0 | 9,27 | 0,24 % | 0,15 % | 0,65 % | 0,24 % |
| 5329/01/82 Produkt t=0,83 h | 9,01 | 0,43 % | 0,07 % | 0,25 % | 0,06 % |
| 5329/01/82 Konzentrat t=0 h | 0,0 | 0,0% | 0,2 % | 0,0 % | 0,0 % |
| 5329/01/82 Konzentrat t=0,83 | h 0,015 | 0,02 % | 0,47 % | 0,71 % | 0,29 % |

Daraus berechnen sich Abreicherungen von 51 % für Natrium, 66 % für Ammonium und 61 % für Kalium. Die mittlere Stromausbeute betrug 82 %. Der Verlust von Pantothensäure in die beiden angrenzenden Kreisläufe lag jeweils bei 0,08 % der eingesetzten Stoffmenge. Der pH-Wert des Produktkreislaufs lag während des Versuchs zwischen pH=7-7,5, im Konzentrat und CaCl₂ Kreislauf bei etwa pH=6. Der Gesamtspannungsabfall stieg im Laufe des Versuchs entsprechend der abnehmenden Leitfähigkeit im CaCl₂ Kreislauf von circa 11 auf 16 V an.

Die so durch Elektrodialyse behandelte Lösung wurde anschließend erfolgreich zum Endprodukt sprühgetrocknet. Hiermit ist gezeigt, daß bei entsprechender Vorbehandlung eine Dreikreiselektrodialyse mit einer monoselektiven Kationenaustauschermembran zum Ionenaustausch von einwertigen gegen zweiwertige Kationen eingesetzt werden kann, um die Sprühtrockenbarkeit des Endprodukts zu erhöhen.

## Patentansprüche

1. Verfahren zur Herstellung von D-Pantothensäure und/oder deren Salze, **dadurch gekennzeichnet, daß**
a) wenigstens ein D-Pantothensäure produzierender Mikroorganismus eingesetzt wird, dessen Pantothensäure-(pan)- und/oder Isoleucin/Valin-(ilv)-Biosynthese dereguliert ist und der wenigstens 2 g/l an Salzen der D-Pantothensäure durch Fermentation in einem Kulturmedium bildet, wobei dem Kulturmedium 0 - 20 g/l freies β-Alanin und/oder β-Alanin-Salz zugeführt wird,
b) die D-Pantothenat enthaltende Fermentationslösung durch Anlegen eins elektrischen Feldes über eine oder mehrere ionenselektive Membran(en) geleitet wird, wobei niedermolekulare Ionen aus der D-Pantothenat-haltigen Lösung entfernt werden,
c) die in der Lösung enthaltene freie D-Pantothensäure durch die Zugabe einer Calcium- und/oder Magnesiumbase auf einen pH-Wert von 3-10 eingestellt wird, wobei eine Lösung erhalten wird, die Calcium- und/oder Magnesium-Pantothenat enthält und
d) die Calcium- und/oder Magnesium-Pantothenat enthaltende Lösung einer Trocknung und/oder Formulierung unterzogen wird.

2. Verfahren gemäß Anspruch 1, **dadurch gekennzeichnet, daß** dem Kulturmedium kein freies β-Alanin und/oder β-Alanin-Salz zugeführt wird.

3. Verfahren gemäß einem der Ansprüche 1 oder 2, **dadurch gekennzeichnet, daß** als D-Pantothensäure produzierender Organismus ein Bakterium, eine Hefe oder ein Pilz eingesetzt wird.

4. Verfahren gemäß einem der Ansprüche 1 bis 3, **dadurch gekennzeichnet, daß** als Mikroorganismus ein Bakterium aus der Familie der Bacillaceae eingesetzt wird.

5. Verfahren gemäß einem der Ansprüche 1 bis 4, **dadurch gekennzeichnet, daß** ein Bakterium der Gattung Bacillus und bevorzugt der Art B. subtils, B. licheniformis oder B. amyloliquefaciens eingesetzt wird.

6. Verfahren gemäß einem der Ansprüche 1 bis 5, **dadurch gekennzeichnet, daß** in Schritt a) ein Gehalt an D-Pantothensäure und/oder deren Salzen von wenigstens 10 g/l Kulturmedium, bevorzugt wenigstens 20 g/l, besonders bevorzugt wenigstens 40 g/l und höchst bevorzugt von wenigstens 60 g/l Kulturmedium gebildet wird.

7. Verfahren gemäß einem der Ansprüche 1 bis 6, **dadurch gekennzeichnet, daß** in Schritt c) der pH der Lösung auf einen Wert von 5-10 eingestellt wird.

8. Verfahren gemäß einem der Ansprüche 1 bis 7, **dadurch gekennzeichnet, daß** in Schritt c) der pH der Lösung auf einen Wert von 5-9, bevorzugt von 6-9 und besonders bevorzugt von 6-8 eingestellt wird.

9. Verfahren gemäß einem der Ansprüche 1 bis 8, **dadurch gekennzeichnet, daß** in Schritt b) monopolare und/oder bipolare Ionenaustausch-Membranen eingesetzt werden.

10. Verfahren gemäß einem der Ansprüche 1 bis 9, **dadurch gekennzeichnet, daß** in Schritt b) monopolare Ionenaustausch-Membranen eingesetzt werden.

11. Verfahren gemäß einem der Ansprüche 1 bis 10, **dadurch gekennzeichnet, daß** in Schritt b) monoselektive Ionenaustausch-Membranen eingesetzt werden.

12. Verfahren gemäß einem der Ansprüche 1 bis 11, **dadurch gekennzeichnet, daß** der pH-Wert der D-Pantothensäure enthaltenden Fermentationslösung vor der Aufbereitung in Schritt b) auf den isoelektrischen pH-Wert der D-Pantothensäure eingestellt wird.

13. Verfahren gemäß einem der Ansprüche 1 bis 12, **dadurch gekennzeichnet, daß** in Schritt b) Stromdichten 1-100 mA/cm², bevorzugt 10-80 mA/cm² und besonders bevorzugt von 20-40 mA/cm² für monopolare Ionentausch-Membranen eingestellt werden.

14. Verfahren gemäß einem der Ansprüche 1 bis 13, **dadurch gekennzeichnet, daß** in Schritt b) Stromdichten 1-150 mA/cm², bevorzugt 30-100 mA/cm² und besonders bevorzugt von 70-90 mA/cm² für bipolare Ionentausch-Membranen eingestellt werden.

15. Verfahren gemäß einem der Ansprüche 1 bis 14, **dadurch gekennzeichnet, daß** in Schritt b) der Gehalt an einwertigen Kationen, bevorzugt Ammonium-, Kalium- und/oder Natrium-Ionen, auf eine Konzentration von ≤ 1g/kg Lösung reduziert wird.

16. Verfahren gemäß einem der Ansprüche 1 bis 15, **dadurch gekennzeichnet, daß** der Lösung in Schritt c) zur Neutralisation Calciumhydroxid, Calciumcarbonat, Calciumoxid, Magnesiumhydroxid und/oder basisches Magnesiumcarbonat in Form eines Feststoffes und/oder als wässrige Suspension zugegeben wird.

17. Verfahren gemäß einem der Ansprüche 1 bis 16, **dadurch gekennzeichnet, daß** der Lösung in Schritt c) eine wässrige Suspension enthaltend 2-55 Gew.-%, bevorzugt 10-50 Gew.-% und besonders bevorzugt 20-40 Gew.-% an Calciumhydroxid zugegeben wird.

18. Verfahren gemäß einem der Ansprüche 1 bis 17 **dadurch gekennzeichnet, daß** der Lösung in Schritt c) eine wässrige Suspension enthaltend 2-65 Gew.-%, bevorzugt 10-50 Gew.-% und besonders bevorzugt 20-40 Gew.-% an Calciumcarbonat zugegeben wird.

19. Verfahren gemäß einem der Ansprüche 1 bis 18, **dadurch gekennzeichnet, daß** der Lösung in Schritt c) eine wässrige Suspension enthaltend 2-60 Gew.-%, bevorzugt 10-50 Gew.-% und besonders bevorzugt 20-40 Gew.-% an Magnesiumhydroxid zugegeben wird.

20. Verfahren gemäß einem der Ansprüche 1 bis 19, **dadurch gekennzeichnet, daß** der Lösung in Schritt c) eine wässrige Suspension enthaltend 2-25 Gew.-%, bevorzugt 10-20 Gew.-% an basischem Magnesiumcarbonat zugegeben wird.

21. Verfahren gemäß einem der Ansprüche 1 bis 20, **dadurch gekennzeichnet, daß** in Schritt c) oder d) eine Suspension erhalten oder vorgelegt wird, die Calcium- und/oder Magnesium-Pantothenat enthält.

## Claims

1. A process for preparing D-pantothenic acid and/or salts thereof which comprises
a) using at least one microorganism which produces D-pantothenic acid and in which the biosynthesis of pantothenic acid (pan) and/or isoleucine/valine (ilv) is deregulated and which forms at least 2 g/l of salts of D-pantothenic acid by fermentation in a culture medium, 0 - 20 g/l of free β-alanine and/or β-alanine salt being supplied to the culture medium,
b) passing the D-pantothenate-comprising fermentation solution through one or more ion-selective membrane(s) by applying an electric field, low-molecular-weight ions being removed from the D-pantothenate-containing solution,
c) adding a calcium base and/or magnesium base to set the free D-pantothenic acid comprised in the solution to a pH of 3-10, a solution being obtained which contains calcium and/or magnesium pantothenate and
d) subjecting the calcium pantothenate- and/or magnesium pantothenate-comprising solution to drying and/or formulation.

2. The process according to claim 1, wherein no free β-alanine and/or β-alanine salt is fed to the culture medium.

3. The process according to either one of claims 1 and 2, wherein the D-pantothenic-acid-producing organism used is a bacterium, a yeast or a fungus.

4. The process according to any one of claims 1 to 3, wherein the microorganism used is a bacterium from the Bacillaceae family.

5. The process according to any one of claims 1 to 4, wherein a bacterium of the genus Bacillus and preferably the species B. subtils, B. licheniformis or B. amyloliquefaciens, is used.

6. The process according to any one of claims 1 to 5, wherein, in step a) a content of D-pantothenic acid and/or salts thereof of at least 10 g/l of culture medium, preferably at least 20 g/l, particularly preferably at least 40 g/l and very highly preferably at least 60 g/l of culture medium is formed.

7. The process according to any one of claims 1 to 6, wherein, in step c), the pH of the solution is set to 5-10.

8. The process according to any one of claims 1 to 7, wherein, in step c), the pH of the solution is set to 5-9, preferably 6-9, and particularly preferably 6-8.

9. The process according to any one of claims 1 to 8, wherein, in step b), monopolar and/or bipolar ion-exchange membranes are used.

10. The process according to any one of claims 1 to 9, wherein, in step b), monopolar ion-exchange membranes are used.

11. The process according to any one of claims 1 to 10, wherein, in step b), monoselective ion-exchange membranes are used.

12. The process according to any one of claims 1 to 11, wherein the pH of the D-pantothenic-acid- comprising fermentation solution, before the treatment in step b), is set to the isoelectric pH of D-pantothenic acid.

13. The process according to any one of claims 1 to 12, wherein, in step b), current densities of 1-100 mA/cm², preferably 10-80 mA/cm², and particularly preferably 20-40 mA/cm², are set for monopolar ion-exchange membranes.

14. The process according to any one of claims 1 to 13, wherein, in step b), current densities of 1-150 mA/cm², preferably 30-100 mA/cm², and particularly preferably 70-90 mA/cm², are set for bipolar ion-exchange membranes.

15. The process according to any one of claims 1 to 14, wherein, in step b), the content of monovalent cations, preferably ammonium, potassium and/or sodium ions, is reduced to a concentration of ≤ 1 g/kg of solution.

16. The process according to any one of claims 1 to 15, wherein, for neutralization, calcium hydroxide, calcium carbonate, calcium oxide, magnesium hydroxide and/or basic magnesium carbonate is added in the form of a solid and/or as aqueous suspension to the solution in step c).

17. The process according to any one of claims 1 to 16, wherein an aqueous suspension comprising 2-55% by weight, preferably 10-50% by weight, and particularly preferably 20-40% by weight, of calcium hydroxide is added to the solution in step c) .

18. The process according to any one of claims 1 to 17, wherein an aqueous suspension comprising 2-65% by weight, preferably 10-50% by weight, and particularly preferably 20-40% by weight, of calcium carbonate is added to the solution in step c).

19. The process according to any one of claims 1 to 18, wherein an aqueous suspension comprising 20-60% by weight, preferably 10-50% by weight, and particularly preferably 20-40% by weight, of magnesium hydroxide is added to the solution in step c).

20. The process according to any one of claims 1 to 19, wherein an aqueous suspension comprising 2-25% by weight, preferably 10-20% by weight, of basic magnesium carbonate is added to the solution in step c).

21. The process according to any one of claims 1 to 20, wherein, in step c) or d), a suspension is obtained or charged which contains calcium pantothenate and/or magnesium pantothenate.

## Revendications

1. Procédé de préparation d'acide D-pantothénique et/ou de sels de ce dernier, **caractérisé en ce que** :
a) on utilise au moins un microorganisme qui produit de l'acide D-pantothénique dont la biosynthèse de l'acide pantothénique (PAN) et/ou de l'isoleucine/valine (ILV) est dérégulée et qui forme au moins 2 g/l de sels d'acide D-pantothénique par fermentation dans un milieu de culture, de la β-alanine libre et/ou un sel de β-alanine étant ajoutés au milieu de culture à raison de 0 à 20 g/l,
b) par application d'un champ électrique, la solution de fermentation qui contient du D-pantothénate est amenée sur une ou plusieurs membranes sélectives vis-à-vis d'ions, les ions à bas poids moléculaire étant ainsi extraits de la solution qui contient du D-pantothénate,
c) l'acide D-pantothénique libre qui est contenu dans la solution est ajusté à un pH d'une valeur de 3 à 10 par addition d'une base calcique et/ou magnésique de manière à obtenir une solution qui contient du pantothénate de calcium et/ou de magnésium et
d) la solution qui contient du pantothénate de calcium et/ou de magnésium subit un séchage et/ou une formulation.

2. Procédé selon la revendication 1, **caractérisé en ce qu'**au milieu de culture, on n'ajoute pas de β-alanine libre et/ou de sel de β-alanine.

3. Procédé selon l'une des revendications 1 ou 2, **caractérisé en ce que** comme organisme qui produit de l'acide D-pantothénique, on utilise une bactérie, une levure ou un champignon.

4. Procédé selon l'une des revendications 1 à 3, **caractérisé en ce que** comme microorganisme, on utilise une bactérie de la famille des Bacillacées.

5. Procédé selon l'une des revendications 1 à 4, **caractérisé en ce que** l'on utilise une bactérie du type Bacillus et de préférence du type Bacillus subtilis, Bacillus licheniformis ou Bacillus amyloliquefaciens.

6. Procédé selon l'une des revendications 1 à 5, **caractérisé en ce qu'**à l'étape a), on forme une teneur en acide D-pantothénique et/ou en ses sels d'au moins 10 g/l de milieu de culture, de préférence d'au moins 20 g/l, de façon particulièrement préférable d'au moins 40 g/l et de la façon la plus préférable d'au moins 60 g/l de milieu de culture.

7. Procédé selon l'une des revendications 1 à 6, **caractérisé en ce qu'**à l'étape c), on règle le pH de la solution à une valeur comprise entre 5 et 10.

8. Procédé selon l'une des revendications 1 à 7, **caractérisé en ce qu'**à l'étape c), on règle le pH de la solution à une valeur de 5 à 9, de préférence de 6 à 9 et de façon particulièrement préférable de 6 à 8.

9. Procédé selon l'une des revendications 1 à 8, **caractérisé en ce qu'**à l'étape b), on utilise des membranes d'échange d'ions monopolaires et/ou bipolaires.

10. Procédé selon l'une des revendications 1 à 9, **caractérisé en ce qu'**à l'étape b), on utilise des membranes d'échange d'ions monopolaires.

11. Procédé selon l'une des revendications 1 à 10, **caractérisé en ce qu'**à l'étape b), on utilise des membranes d'échange d'ions monosélectives.

12. Procédé selon l'une des revendications 1 à 11, **caractérisé en ce qu'**avant la préparation de l'étape b), la valeur du pH de la solution de fermentation qui contient de l'acide D-pantothénique est ajusté à la valeur isoélectrique du pH de l'acide D-pantothénique.

13. Procédé selon l'une des revendications 1 à 12, **caractérisé en ce qu'**à l'étape b), on règle des densités de courant de 1 à 100 mA/cm², de préférence de 10 à 80 mA/cm² et de façon particulièrement préférable de 20 à 40 mA/cm² pour les membranes d'échange d'ions monopolaires.

14. Procédé selon l'une des revendications 1 à 13, **caractérisé en ce qu'**à l'étape b), on règle des densités de courant de 1 à 150 mA/cm², de préférence de 30 à 100 mA/cm² et de façon particulièrement préférable de 70 à 90 mA/cm² pour les membranes d'échange d'ions bipolaires.

15. Procédé selon l'une des revendications 1 à 14, **caractérisé en ce qu'**à l'étape b), on réduit la teneur en cations monovalents et de préférence en ions ammonium, potassium et/ou sodium à une concentration inférieure ou égale à 1 g/kg de solution.

16. Procédé selon l'une des revendications 1 à 15, **caractérisé en ce qu'**à l'étape c), on ajoute à la solution pour la neutraliser de l'hydroxyde de calcium, du carbonate de calcium, de l'oxyde de calcium, de l'hydroxyde de magnésium et/ou du carbonate basique de magnésium sous forme solide et/ou d'une suspension aqueuse.

17. Procédé selon l'une des revendications 1 à 16, **caractérisé en ce qu'**à l'étape c), on ajoute à la solution une suspension aqueuse qui contient de 2 à 55 % en poids, de préférence de 10 à 50 % en poids et de façon particulièrement préférable de 20 à 40 % en poids d'hydroxyde de calcium.

18. Procédé selon l'une des revendications 1 à 17, **caractérisé en ce qu'**à l'étape c), on ajoute à la solution une suspension aqueuse qui contient de 2 à 65 % en poids, de préférence de 10 à 50 % en poids et de façon particulièrement préférable de 20 à 40 % en poids de carbonate de calcium.

19. Procédé selon l'une des revendications 1 à 18, **caractérisé en ce qu'**à l'étape c), on ajoute à la solution une suspension aqueuse qui contient de 2 à 60 % en poids, de préférence de 10 à 50 % en poids et de façon particulièrement préférable de 20 à 40 % en poids d'hydroxyde de magnésium.

20. Procédé selon l'une des revendications 1 à 19, **caractérisé en ce qu'**à l'étape c), on ajoute à la solution une suspension aqueuse qui contient de 2 à 25 % en poids et de préférence de 10 à 20 % en poids de carbonate basique de magnésium.

21. Procédé selon l'une des revendications 1 à 20, **caractérisé en ce qu'**à l'étape c) ou à l'étape d), on obtient ou on prépare une suspension qui contient du pantothénate de calcium et/ou de magnésium.
